(19) Europäisches Patentamt
European Patent Office
Office européen des brevets

(11) **EP 4 186 514 A1**

(12) **EUROPEAN PATENT APPLICATION**

(43) Date of publication:
**31.05.2023 Bulletin 2023/22**

(21) Application number: **21210303.0**

(22) Date of filing: **24.11.2021**

(51) International Patent Classification (IPC):
***A61K 31/7076*** *(2006.01)* ***A61K 9/00*** *(2006.01)*
***A61P 1/16*** *(2006.01)*

(52) Cooperative Patent Classification (CPC):
**A61K 31/7076; A61P 1/16**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Future Medicine Co., Ltd.**
**Seongnam-si, Gyeonggi-do 13449 (KR)**

(72) Inventors:
- **LEE, Hyuk Woo**
  **13449 Gyeonggi-do (KR)**
- **AHN, Sang Yeop**
  **13449 Gyeonggi-do (KR)**
- **CHO, Hyeon Deok**
  **13449 Gyeonggi-do (KR)**
- **CHOI, Yoon Pyo**
  **13449 Gyeonggi-do (KR)**
- **SEO, Seong Wook**
  **13449 Gyeonggi-do (KR)**

(74) Representative: **Roos, Peter**
**c/o Roospatent**
**Noldinstrasse 7**
**81545 München (DE)**

Remarks:
The complete document including Reference Table(s) and the Sequence Listing(s) can be downloaded from the EPO website

(54) **PHARMACEUTICAL COMPOSITION FOR PREVENTING OR TREATING CHOLANGITIS OR LIVER DISEASE CAUSED BY CHOLANGITIS COMPRISING ADENOSINE DERIVATIVE**

(57) The present invention relates to a pharmaceutical composition comprising an adenosine derivative which can be usefully used for preventing or treating cholangitis or a liver disease caused by cholangitis, and the composition comprises a compound represented by Chemical Formula 1 or a pharmaceutically acceptable salt thereof and a pharmaceutically acceptable carrier. Chemical Formula 1 is described in detail in the specification.

[Fig. 1]

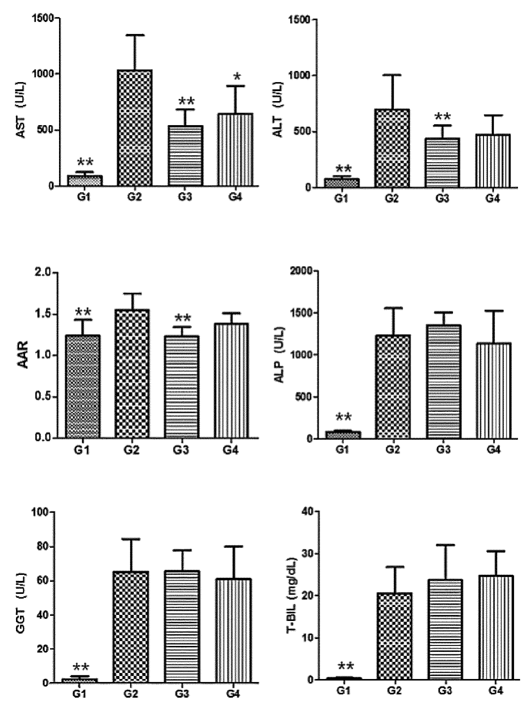


EP 4 186 514 A1

SEQUENCE LISTING

<110> Future Medicine Co., Ltd.

<120> PHARMACEUTICAL COMPOSITION FOR PREVENTING OR TREATING CHOLANGITIS OR LIVER DISEASE CAUSED BY CHOLANGITIS COMPRISING ADENOSINE DERIVATIVE

<130> 16132 P 2339 EP

<160> 8

<170> PatentIn version 3.2

<210> 1
<211> 20
<212> DNA
<213> Acta2 (NM_007392.3) F

<400> 1
tatgctaaca acgtcctgtc 20

<210> 2
<211> 20
<212> DNA
<213> Acta2 (NM_007392.3) R

<400> 2
agacagagta cttgcgttct 20

<210> 3
<211> 20
<212> DNA
<213> Col1a1 (NM_007742.4) F

<400> 3
gaacctaacc atctggcatc 20

<210> 4
<211> 20
<212> DNA
<213> Col1a1 (NM_007742.4) R

<400> 4
aatagaacgg tctctcccac 20

<210> 5
<211> 20
<212> DNA
<213> Timp1 (NM_001044384.1) F

<400> 5
agacacacca gagcagatac 20

<210> 6
<211> 20
<212> DNA
<213> Timp1 (NM_001044384.1) R

<400> 6
cgctggtata aggtggtctc 20

<210> 7
<211> 19
<212> DNA
<213> Gapdh (NM_001289726.1) F

<400> 7
tgcaccacca actgcttag 19

<210> 8
<211> 19
<212> DNA
<213> Gapdh (NM_001289726.1) R

<400> 8
ggatgcaggg atgatgttc 19

## Description

[Technical Field]

The present invention relates to a pharmaceutical composition comprising an adenosine derivative which can be usefully used for preventing or treating cholangitis or a liver disease caused by cholangitis.

[Background Art]

Cholangitis is an acute or chronic inflammatory disease that occurs in the bile duct system, and may be caused by stagnant bile when the lower biliary tract is closed by inflammation in the bile ducts. The accumulation of specific bile acids in the liver in a cholestatic environment causes an inflammatory response which may induce liver injury.

In particular, primary biliary cholangitis (PBC) or primary biliary cirrhosis, which is a rare disease as a type of cholangitis, is the most common liver disease associated with chronic cholecystitis in adults as an autoimmune chronic liver disease that affects bile secretion. PBC is characterized by damage to epithelial cells called bile duct cells which surround the hepatic bile duct, and damage to the bile duct causes cholestasis, and when bile accumulates in the liver, an inflammatory response occurs, thereby inducing liver injury such as liver fibrosis and liver cirrhosis.

Currently, disclosed therapeutic agents for PBC include ursodeoxycholic acid (UDCA) and obeticholic acid (OCA), and both of them are synthetic bile acids used to prevent inflammatory cholestatic injury in bile duct cells. However, up to 50% of patients do not experience any effect after 4 months of UDCA treatment, and OCA may induce acute liver failure in patients with liver cirrhosis, so that its use in patients with end-stage liver injury is limited.

Meanwhile, adenosine generally accumulates in stress areas and interacts with adenosine receptors (ARs), and such adenosine receptors are known as G-binding protein receptors whose expression appear to be upregulated in inflammatory liver tissue. Since the downstream components of the $A_3$ adenosine receptor ($A_3$AR) regulatory pathway can contribute to the inflammatory and fibrotic pathways, the adenosine pathway may be associated with the fibrotic and inflammatory aspects of PBC.

Therefore, the present inventors studied an $A_3$AR antagonist and agonist as an agent for preventing and treating cholangitis such as PBC for the first time, and discovered that a specific adenosine derivative compound is effective for cholangitis and liver injury caused by cholangitis, thereby completing the present invention.

[Related Art Document]

[Non-Patent Documents]

Liedtke, C., et al. Experimental liver fibrosis research: update on animal models, legal issues and translational aspects. Fibrogenesis Tissue Repair. 6, (1), 19 (2013).

Li, X., Liu, R., Huang, Z., Gurley, E.C., Wang, X., Wang, J., He, H., Yang, H., Lai, G, Zhang, L., Bajaj, J.S., White, M., Pandak, W.M., Hylemon, P.B. and Zhou, H. (2018), Cholangiocyte-derived exosomal long noncoding RNA H19 promotes cholestatic liver injury in mouse and humans. Hepatology, 68: 599-615. doi:10.1002/hep.29838

You Li, Ruqi Tang, Patrick S.C. Leung, M. Eric Gershwin, Xiong Ma, Bile acids and intestinal microbiota in autoimmune cholestatic liver diseases, Autoimmunity Reviews, Volume 16, Issue 9, 2017, Pages 885-896, ISSN 1568-9972, https://doi.org/10.1016/j.autrev.2017.07.002.

Lleo, A., Maroni, L., Glaser, S., Alpini, G, & Marzioni, M. (2014). Role of cholangiocytes in primary biliary cirrhosis. Semin Liver Dis, 34(3), 273-284. doi:10.1055/s-0034-1383727

Civan, J. (2019). Primary Biliary Cholangitis. Merck Manual Professional Version.

Aguilar, M. T., & Chascsa, D. M. (2020). Update on Emerging Treatment Options for Primary Biliary Cholangitis. Hepat Med, 12, 69-77. doi:10.2147/HMER.S205431

[Disclosure]

[Technical Problem]

Therefore, a problem to be solved by the present invention is to provide a pharmaceutical composition comprising an adenosine derivative capable of preventing or treating cholangitis or a liver disease caused by cholangitis.

The problems of the present invention are not limited to the aforementioned technical problems, and other technical problems, which have not been mentioned, may be clearly understood by a person with ordinary skill in the

art from the following description.

[Technical Solution]

The pharmaceutical composition for preventing and/or treating cholangitis or a liver disease caused by cholangitis according to an embodiment of the present invention to solve the problem comprise a compound represented by the following Chemical Formula 1 or a pharmaceutically acceptable salt thereof; and a pharmaceutically acceptable carrier.

<Chemical Formula 1>

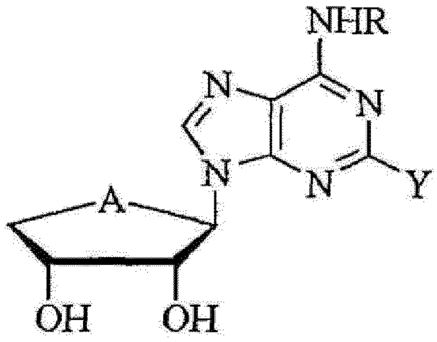

wherein A is O or S, R is an unsubstituted $C_1$ to $C_5$ alkyl; a $C_1$ to $C_5$ alkyl substituted with at least one $C_6$ to $C_{10}$ aryl; unsubstituted benzyl; benzyl substituted with one or more selected from the group consisting of a halogen and a $C_1$ to $C_4$ alkoxy; or benzyl substituted with hydroxycarbonyl, and Y is H or a halogen element.

In Chemical Formula 1, A may be O or S, R may be methyl; ethyl; propyl; naphthylmethyl; benzyl; benzyl substituted with one or more selected from the group consisting of a halogen and a $C_1$ to $C_3$ alkoxy; or toluic acid, and Y may be H or Cl.

In Chemical Formula 1, A may be S, R may be methyl, ethyl, 1-naphthylmethyl, benzyl, 2-chlorobenzyl, 3-fluorobenzyl, 3-chlorobenzyl, 3-bromobenzyl, 3-iodobenzyl, 2-methoxy-5-chlorobenzyl, 2-methoxybenzyl or 3-toluic acid, and Y may be Cl.

Chemical Formula 1 may be the following formula A.

<formula A>

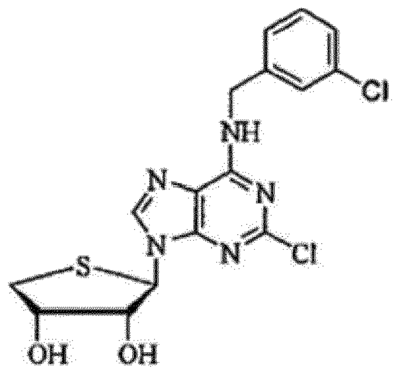

The cholangitis may be a cholestatic disease (cholestasis).

The cholestatic disease may include one or more selected from the group consisting of primary biliary cholangitis (PBC), primary sclerosing cholangitis (PSC), cholecystitis, drug induced cholestasis, post-liver transplantation cholestasis, progressive familial intrahepatic cholestasis (PFIC), Alagille Syndrome (ALGS), biliary atresia, intrahepatic cholestasis of pregnancy (ICP), cholelithiasis, infectious cholangitis, cholangitis associated with Langerhans cell histiocytosis, non-syndromic ductal paucity and total parenteral nutrition-associated cholestasis.

The cholestatic disease further includes a complication caused by the cholestatic disease, and the complication may include one or more selected from the group consisting of pruritus, hypercholesterolemia, Sicca syndrome, Raynaud

syndrome, osteoporosis, ulcerative colitis and colorectal cancer.

The liver disease caused by cholangitis may include one or more of liver fibrosis, liver cirrhosis and hepatitis.

It is possible to lower the values of one or more of AST, ALT, AAR, hyaluronic acid and hydroxyproline in a liver tissue or blood of an individual to which the composition is administered.

The pharmaceutical composition may be formulated into an oral administration agent.

The oral administration agent may further comprise methyl cellulose (MC).

The oral administration agent may be an oral administration agent in which the compound represented by Chemical Formula 1 or a pharmaceutically acceptable salt thereof is filled in a powder state in a capsule.

Specific details of other embodiments are included in the detailed description.

[Advantageous Effects]

Since the adenosine derivative according to the embodiments of the present invention has an effect of ameliorating cholangitis and liver injury such as liver fibrosis caused by the cholangitis, a pharmaceutical composition containing such an adenosine derivative can be used as an agent for preventing or treating cholangitis or a liver disease caused by cholangitis.

Further, since the pharmaceutical composition has excellent drug absorption during oral administration, is biocompatible due to little in vivo toxicity, and has excellent storage stability during formulation as an oral administration agent, the pharmaceutical composition can be used as an oral administration agent for preventing or treating cholangitis or a liver disease caused by cholangitis.

The effects according to the embodiments of the present invention are not limited to the contents exemplified above, and more various effects are included in the present specification.

[Description of Drawings]

FIG. 1 is a set of graphs showing the results of measuring serum ALT, AST, ALP, GGT and T-BIL of the experimental animal G1 to G4 groups in Experimental Example 1.

FIG. 2 is a set of graphs showing the results of measuring the expression levels of Acta2, Col1a1 and Timp1 mRNA of the experimental animal G1 to G4 groups in Experimental Example 1.

FIG 3 is a set of graphs showing the results of measuring the levels of TNF-$\alpha$, IL-1$\beta$ and hyaluronic acid of the experimental animal G1 to G4 groups in Experimental Example 1.

FIG 4 is a graph showing the results of measuring the hydroxyproline values of the experimental animal G1 to G4 groups in Experimental Example 1.

FIG 5 is a graph obtained from the blood concentration-time data of Experimental Example 3.

FIG 6 is a graph obtained from the blood concentration-time data of Experimental Example 4.

FIG 7 is a graph obtained from the blood concentration-time data of Experimental Example 5.

FIG 8 is a graph obtained from the blood concentration-time data of Experimental Example 6.

FIG 9 is a graph obtained from the blood concentration-time data of Experimental

Example 7.

FIGS. 10 to 13 are views showing the anti-inflammatory activity of the adenosine derivative of the present invention according to animal experiments.

[Modes of the Invention]

The benefits and features of the present application, and the methods of achieving the benefits and features will become apparent with reference to embodiments to be described below in detail. However, the present invention is not limited to the embodiments to be disclosed below and may be implemented in various other forms, and the embodiments are only provided for rendering the disclosure of the present invention complete and for fully representing the scope of the invention to a person with ordinary skill in the technical field to which the present invention pertains, and the present invention will be defined only by the scope of the claims.

The terms used in the present specification are used merely to describe embodiments, and are not intended to limit the present invention. In the present specification, the 'and/or' includes each and all combinations of one or more of the items mentioned. Further, the singular form includes the plural forms unless specifically stated in a phrase. The terms 'comprises' and/or 'comprising' used in the specification do not exclude the presence or addition of one or more

other constituent elements in addition to the referenced constituent elements. The numerical range indicated by using '-' or 'to' indicates a numerical range including values described before and after it as a lower limit and an upper limit, respectively, unless otherwise stated. 'About' or 'approximately' means a value or numerical range within 20% of the value or numerical range described thereafter.

Further, in describing the constituent elements of the examples of the present invention, terms such as first, second, A, B, (a), and (b) may be used. These terms are merely for distinguishing one constituent element from another, and the nature, turn, or order of the corresponding constituent element is not limited by the term.

Unless otherwise defined, all the terms (including technical and scientific terms) used in the present specification will be able to be used as a meaning which may be commonly understood to a person with ordinary skill in the technical field to which the present invention pertains. In addition, the terms defined in a dictionary generally used are not interpreted ideally or excessively unless the terms have been clearly and specially defined.

Moreover, in describing the examples of the present invention, when it is determined that the specific description of relevant known configurations or functions obstructs the understanding for the examples of the present invention, the detailed description thereof will be omitted.

As used herein, the term "pharmaceutically acceptable salt" means a salt prepared according to a conventional method in the art, and such preparation methods are known to those skilled in the art. Specifically, the pharmaceutically acceptable salts include salts derived from the following inorganic and organic acids and bases which are pharmacologically or physiologically acceptable, but are not limited thereto. Examples of a suitable acid may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, and the like. Salts derived from a suitable base may include alkali metals, such as sodium or potassium, alkaline earth metals such as magnesium.

As used herein, 'prevention' refers to suppressing or delaying the onset of a symptom or disease in an individual who does not yet have the symptom or disease, but may suffer from the symptom or disease.

As used herein, 'treatment' refers to all actions that ameliorate or beneficially change a symptom from an individual, and refers to, for example, (a) suppression of the development (exacerbation) of a symptom or disease, (b) reduction or amelioration of a symptom or disease, or (c) elimination of a symptom or disease.

As used herein, 'individual' refers to an animal, particularly a mammal, including a human having a symptom or disease that can be 'prevented' or 'treated' by administering the composition of the present invention.

As used herein, 'substituted $C_n$ to $C_{n+m}$' compound includes a case where the number of all carbons of a compound including a substituted moiety is n to n+m, as well as a case where the number of carbons of the compound except for the substituted moiety is n to n+m.

Hereinafter, the present invention will be described in detail.

The present invention provides a pharmaceutical composition for preventing and/or treating cholangitis or a liver disease caused by cholangitis, comprising a compound of the following Chemical Formula 1 or a pharmaceutically acceptable salt thereof.

<Chemical Formula 1>

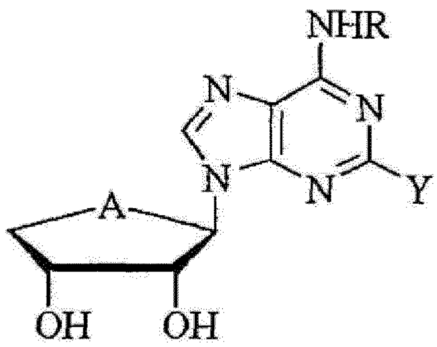

wherein,

A is O or S,
R is an unsubstituted $C_1$ to $C_5$ alkyl; a $C_1$ to $C_5$ alkyl substituted with at least one $C_6$ to $C_{10}$ aryl; unsubstituted benzyl; benzyl substituted with one or more selected from the group consisting of a halogen and a $C_1$ to $C_4$ alkoxy; or benzyl substituted with hydroxycarbonyl), and
Y is H or a halogen element.

Preferably,

A is O or S,
R is methyl; ethyl; propyl; napthylmethyl; benzyl; benzyl substituted with one or more selected from the group consisting of a halogen and a $C_1$ to $C_3$ alkoxy; or toluic acid, and
Y is H or Cl.

More preferably,

Ais S,
R is methyl, ethyl, 1-naphthylmethyl, benzyl, 2-chlorobenzyl, 3-fluorobenzyl, 3-chlorobenzyl, 3-bromobenzyl, 3-iodobenzyl, 2-methoxy-5-chlorobenzyl, 2-methoxybenzyl or 3-toluic acid, and
Y is Cl.

The adenosine derivative according to the present invention is the compound represented by Chemical Formula 1, and preferred examples thereof are as follows.

1) (2R,3R,4S)-2-(2-chloro-6-(3-fluorobenzylamino)-9H-purin-9-yl)tetrahydrothiophen-3,4-diol;
2) (2R,3R,4S)-2-(2-chloro-6-(3-chlorobenzylamino)-9H-purin-9-yl)tetrahydrothiophen-3,4-diol;
3) (2R,3R,4S)-2-(6-(3-bromobenzylamino)-2-chloro-9H-purin-9-yl)tetrahydrothiophen-3,4-diol;
4) (2R,3R,4S)-2-(2-chloro-6-(3-iodobenzylamino)-9H-purin-9-yl)tetrahydrothiophen-3,4-diol;
5) (2R,3R,4S)-2-(2-chloro-6-(2-chlorobenzylamino)-9H-purin-9-yl)tetrahydrothiophen-3,4-diol;
6) (2R,3R,4S)-2-(2-chloro-6-(5-chloro-2-methoxybenzylamino)-9H-purin-9-yl)tetrahydrothiophen-3,4-diol;
7) (2R,3R,4S)-2-(2-chloro-6-(2-methoxybenzylamino)-9H-purin-9-yl)tetrahydrothiophen-3,4-diol;
8) (2R,3R,4S)-2-(2-chloro-6-(naphthalen-1-ylmethylamino)-9H-purin-9-yl)tetrahydrothiophen-3,4-diol;
9) 3-((2-chloro-9-((2R,3R,4S)-3,4-dihydroxytetrahydrothiophen-2-yl)-9H-purine-6-ylamino)methyl)benzoic acid;
10) 2-(2-chloro-6-methylamino-purin-9-yl)tetrahydrothiophen-3,4-diol;
11) (2R,3R,4S)-2-(6-(3-fluorobenzylamino)-9H-purin-9-yl)tetrahydrothiophen-3,4-diol;
12) (2R,3R,4S)-2-(6-(3-chlorobenzylamino)-9H-purin-9-yl)tetrahydrothiophen-3,4-diol;
13) (2R,3R,4S)-2-(6-(3-bromobenzylamino)-9H-purin-9-yl)tetrahydrothiophen-3,4-diol;
14) (2R,3R,4S)-2-(6-(3-iodobenzylamino)-9H-purin-9-yl)tetrahydrothiophen-3,4-diol;
15) (2R,3R,4R)-2-(6-(3-bromobenzylamino)-2-chloro-9H-purin-9-yl)tetrahydrofuran-3,4-diol; or
16) (2R,3R,4R)-2-(2-chloro-6-(3 -iodobenzylamino)-9H-purin-9-yl)tetrahydrofuran-3,4-diol.

Preferred examples of the adenosine derivative represented by Chemical Formula 1 may be (2R,3R,4S)-2-(2-chloro-6-(3-chlorobenzylamino)-9H-purin-9-yl)tetrahydrothiophen-3,4-diol which is a compound represented by the following formula A.

<formula A>

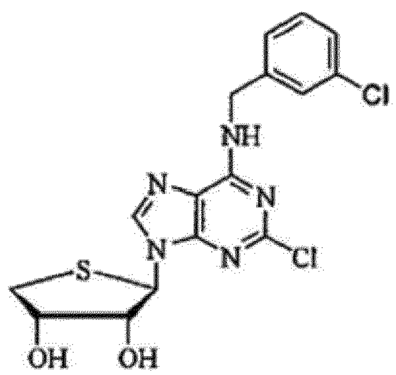

The adenosine derivatives according to the present invention can be synthesized by the method described in Korean Patent No. 10-1396092.

The adenosine derivative represented by Chemical Formula 1 according to the present invention can be used in the form of a pharmaceutically acceptable salt. As the salt, known acid addition salts formed by various organic or

inorganic acids that are pharmaceutically acceptable are useful.

The adenosine derivative represented by Chemical Formula 1 according to the present invention may include not only a pharmaceutically acceptable salt, but also all salts prepared by typical methods, hydrates and solvates thereof.

Further, the pharmaceutical composition of the present invention may further include a pharmaceutically acceptable carrier. The pharmaceutically acceptable carrier refers to any suitable adjuvant, carrier, excipient or stabilizer, and may take the form of a solid or solid such as a tablet, a capsule, a powder, a solution, a suspension or an emulsion. For example, the pharmaceutically acceptable carrier may be a lubricant and a non-active filler, such as a capsule. The tablet, capsule, and the like may contain a binder; an excipient; a disintegrating agent; a lubricant; and a sweetener. When the unit dosage form is a capsule, the capsule may contain a liquid carrier in addition to the above-described type of materials. When administered as an injection, the carrier may be a solvent or dispersion medium containing water, ethanol, a polyol or a suitable mixture thereof.

Cholangitis means an acute or chronic inflammatory disease that occurs in the bile duct system, but is not limited thereto. Cholangitis may specifically include a cholestatic disease (cholestasis), and the cholestatic disease may one or more of primary biliary cholangitis (PBC; or primary biliary cirrhosis), primary sclerosing cholangitis (PSC), cholecystitis, rug induced cholestasis, post-liver transplantation cholestasis, progressive familial intrahepatic cholestasis (PFIC), Alagille Syndrome (ALGS), biliary atresia, intrahepatic cholestasis of pregnancy (ICP), cholelithiasis, infectious cholangitis, cholangitis associated with Langerhans cell histiocytosis, non-syndromic ductal paucity and total parenteral nutrition-associated cholestasis. In addition, the cholestatic disease may also include a complication caused by the cholestatic disease, may include one or more of pruritus, hypercholesterolemia, Sicca syndrome, Raynaud syndrome, and osteoporosis as the complication of PBC, and may also include one or more of ulcerative colitis and colorectal cancer as the complication of PSC.

Furthermore, the liver caused by cholangitis may specifically mean liver injury such as liver fibrosis, liver cirrhosis and/or hepatitis. Particularly in the case of cholangitis such as PBC and PSC, a cholestatic environment is created due to damage to the epithelial cells of the bile duct, and accordingly, bile acids may accumulate in the liver, causing inflammation, fibrosis, and cirrhosis of the liver. the liver caused by cholangitis may comprise, such as NASH (non-alcoholic steatohepatitis), alcoholic steatohepatitis (ASH), liver injury associated with or caused by alcohol consumption in a mammal afflicted with NASH, alcoholic hepatitis, drug induced liver injury, viral hepatitis, toxic liver conditions, etc.

The cholangitis or the liver disease caused by cholangitis may enhance the values of one or more markers of AST, ALT, AAR (AST/ALT), hyaluronic acid and hydroxyproline in a liver tissue or blood of an individual. Therefore, as the cholangitis or the liver disease caused by cholangitis is ameliorated in the individual to which the pharmaceutical composition according to the present invention is administered, one or more values of the markers in the liver tissue or blood may be lowered.

The pharmaceutical composition according to the present invention may be administered systemically or locally, and may be formulated using an excipient (or a diluent) such as a filler, an extender, a binder, a wetting agent, a disintegrating agent, and a surfactant, which may be generally used for administration.

The pharmaceutical composition for preventing and/or treating cholangitis or a liver disease caused by cholangitis of the present invention may be formulated, particularly, as an oral administration agent.

The oral administration agent may comprise the compound represented by Chemical Formula 1 and/or a pharmaceutically acceptable salt and an excipient. The excipient may include one or more selected from the group consisting of methyl cellulose (MC), dimethylsulfoxide (DMSO), polyethylene glycol (PEG), distilled water (DW), a capsule, and the like. A preferred example of the excipient may be 0.5 wt% of methyl cellulose.

The oral administration may be an oral administration agent in which the compound represented by Chemical Formula 1 and/or a pharmaceutically acceptable salt thereof is filled in a powder state or in a state of a solution in which an excipient is dissolved in a capsule.

A preferred dose of the pharmaceutical composition the present invention varies depending on various factors such as the condition and body weight of a patient, the degree of a disease, the form of drug, the administration route, and the duration, but may be appropriately selected by the person skilled in the art. Further, the administration route may be changed depending on the condition of a patient and the severity thereof.

Hereinafter, embodiments of the present invention will be described in detail with reference to Preparation Examples and Experimental Examples, but it is obvious that the effects of the present invention are not limited by the following Experimental Examples.

**Experimental Example 1: Efficacy experiment of adenosine derivative of present invention on cholangitis and liver disease caused by cholangitis**

Experimental method

After 23 C57BL/6 mice were prepared and subjected to quarantine and acclimation, a bile duct ligation (BDL)

surgery and a sham surgery were performed by dividing the animals into groups.

The BDL rodent model is one of the most commonly used methods to induce obstructive cholestatic injury (Tag, C. G et al., Bile Duct Ligation in Mice: Induction of Inflammatory Liver Injury and Fibrosis by Obstructive Cholestasis, <em>J. Vis. Exp.</em> (96), e52438, doi: 10.3791/52438 (2015)). The BDL model induces liver fibrosis and inflammation by causing obstructive cholestasis, and since such symptoms are generally observed in patients with PBC after stage II, the corresponding model may adequately reflect an important aspect of PBC pathology. The advantages of the BDL model are technical validity, reproducibility, and the short time it takes to reach the pathogenomonic status of human portal hypertension.

The surgery day was set to day 0, and the test material was orally administered once a day for 2 weeks from day 1 to day 14. The dose was 10 mL/kg based on the body weight measured before administration. As a test material, a compound of formula A mixed with 0.5% methylcellulose (MC), which is a vehicle, was used, and only the vehicle was used in the control.

The treatment contents of each group are summarized as in the following Table 1.

[Table 1]

| Group | Surgery | Drug | Dose (mg/kg) | Volume (mL/kg) | Route | Period (day) | Mice number |
|---|---|---|---|---|---|---|---|
| G1 | Sham | Vehicle | - | 10 | Oral | 13 | 5 |
| G2 | BDL | Vehicle | - | 10 | Oral | 13 | 6 |
| G3 | BDL | FM101 | 10 | 10 | Oral | 13 | 6 |
| G4 | BDL | FM101 | 30 | 10 | Oral | 13 | 6 |

Blood was collected through the abdominal vein under respiratory anesthesia on day 14, and serum was isolated. After blood collection, liver tissue was collected and weighed, half of the left lateral lobe from the tissue was placed in a tube and immersed in liquid nitrogen, and the other half was fixed in neutral formalin.

- Blood biochemical analysis: aspartate aminotransferase (AST), alanine aminotransferase (ALT), alkaline phosphatase (ALP), $\gamma$-glutamyl transpeptidase (GGT) and total bilirubin (T-BIL) were measured using a serum biochemical analyzer (Accute, Toshiba). In addition, after blood collection, the body weight was measured by collecting liver tissue.

- mRNA expression: RNA was extracted using a portion of the left lateral lobe removed from the liver tissue, cDNA was synthesized using an RNase inhibitor (DR22-R10k, Solgent, Korea) and DiaStar ™ RT Kit, and qRT-PCR was performed using Power SYBR Green PCR Master Mix (Applied Biosystems by Thermo Fisher Scientific, USA).

The following Table 2 lists sequences of primers of mouse Gapdh, Acta2(actin alpha 2 chain), Col1a1(proalpha1(I) chain) and tissue inhibitor of metalloproteinases-1 (Timp1) used for qRT-PCR.

[Table 2]

| Species | Target (Accession No.) | | Sequence | Tm | Product Size (bp) |
|---|---|---|---|---|---|
| Mouse Mus musculus | Acta2 (NM_007392.3) – | F | TATGCTAACAACGTCCTGTC | 55.22 | 139 |
| | | R | AGACAGAGTACTTGCGTTCT | 56.23 | |
| | Col1a1 (NM_ 007742.4) | F | GAACCTAACCATCTGGCATC | 55.89 | 193 |
| | | R | AATAGAACGGTCTCTCCCAC | 56.36 | |
| | Timp1 (NM_ 001044384.1) | F | AGACACACCAGAGCAGATAC | 56.66 | 203 |
| | | R | CGCTGGTATAAGGTGGTCTC | 57.49 | |
| | Gapdh (NM_ 001289726.1) | F | TGCACCACCAACTGCTTAG | 57.98 | 177 |
| | | R | GGATGCAGGGATGATGTTC | 55.73 | |

- Analysis of cytokine levels: TNF-$\alpha$, IL-1$\beta$ and hyaluronic acid levels in serum were measured using an ELISA kit.
- Analysis of hydroxyproline: a portion of the left lateral lobe of liver tissue was extracted, and the hydroxyproline

value of liver lysate was measured by a microplate reader (SpectraMax ABS Plus, Molecular Devices) using a hydroxyproline assay kit (Abcam).

Experimental results

The experimental results are shown as mean ± SD, and the test material-administered group was compared with the vehicle control (BDL, G2). Statistical analysis was performed using one-way ANOVA followed by Fisher's least significant difference test using GraphPad Prism (version 5.01) and SPSS (version 24) software. $P < 0.05$ was considered statistically significant.

1) Blood biochemical analysis

Serum ALT, AST, ALP, GGT and T-BIL were measured to investigate hepatobiliary dysfunction. FIG 1 is a set of graphs showing the results of measuring serum ALT, AST, ALP, GGT and T-BIL of G1 to G4 groups. (In all the graphs, *: significantly different from BDL (G2) (P<0.05), **: significantly different from BDL (G2) (P<0.01))

In the BDL control group (G2), AST, ALT, AAR (AST/ALT ratio), ALP, GGT and T-BIL levels were significantly increased compared to the sham group (G1).

Compared with the BDL control group (G2), ALT and AAR decreased by 37% and 21%, respectively, in the group (G3) to which 10 mg/kg of the compound of the formula A was administered, and AST was also decreased by 48% and 38% in the group (G3) to which 10 mg/kg of the compound of the formula A was administered and the group (G4) to which 30 mg/kg of the compound of the formula A was administered, respectively. Since ALT, AAR and AST are used as markers for liver injury and fibrosis caused by BDL (Afdhal NH, Nunes D. Evaluation of liver fibrosis: concise review. Am J Gastroenterol 2004;99:1160-1174.v), these results show that liver injury and fibrosis were reduced in the BDL groups (G3, G4) to which the compound of the formula A was administered.

2) mRNA expression level

FIG 2 is a set of graphs showing the results of measuring the expression levels of Acta2, Col1a1 and Timp1 mRNA of G1 to G4 groups.

Acta2 mRNA levels were induced in all the BDL groups, and decreased in BDL groups (G3, G4) to which the compound of the formula A was administered. Since hepatic stellate cells (HSCs) regulate fibrosis-promoting factors such as Timp1, Type 1 collagen, Col1a1 and Acta2 in cholangitis, these results show that liver injury and fibrosis were reduced in the BDL groups (G3, G4) treated with the compound of the formula A.

3) Cytokine level

FIG 3 is a set of graphs showing the results of measuring the levels of TNF-$\alpha$, IL-1$\beta$ and hyaluronic acid of G1 to G4 groups.

TNF-$\alpha$ and hyaluronic acid were significantly induced in the BDL control group (G2) compared to the sham group (G1). Hyaluronic acid values were reduced by 51% and 47% in all groups treated with the compound of the formula A (G3, G4), respectively, compared to the BDL control group (G2).

Since the amount of hyaluronic acid in serum significantly correlates with the degree of liver fibrosis (Afdhal NH, Nunes D. Evaluation of liver fibrosis: concise review. Am J Gastroenterol 2004;99:1160-1174.v), these results show that liver fibrosis and cirrhosis were reduced in the BDL groups (G3, G4) treated with the compound of the formula A.

4) Hydroxyproline analysis

FIG 4 is a graph showing the results of measuring the hydroxyproline values of G1 to G4 groups.

The content of liver hydroxyproline was significantly reduced in the BDL control group (G2) compared to the sham group (G1), and was decreased by 39% in the group (G3) to which 10 mg/kg of the compound of the formula A was administered.

Since quantification of hydroxyproline in liver tissue is a method capable of comparing the progression of fibrosis in liver tissue (Arjmand, A. et al., Quantification of Liver Fibrosis-A Comparative Study. Appl. Sci. 2020, 10, 447), these results show that hydroxyproline was suppressed, and thus liver fibrosis was reduced in the BDL group (G3) treated with the compound of the formula A.

As described above, since the adenosine derivative of the present invention can induce improvement of liver function and amelioration of fibrosis by suppressing AST, ALT, AAR (AST/ALT), hyaluronic acid, hydroxyproline, and the like in the BDL mouse model in which liver fibrosis was induced, it can be seen that the adenosine derivative of the

present invention can be utilized as an agent for preventing or treating cholangitis or a liver disease caused by cholangitis.

**<Experimental Example 2> Physicochemical Properties Test of the Adenosine Derivative of the Inventive Concept**

In order to test the physicochemical properties of the adenosine derivative of the inventive concept, experiments were conducted on the compound of the formula A in vitro, and the results are shown in Table 3. Plasma stability and protein binding were measured using rat and human plasma.

[Table 3]

| Physical Properties (ADME Properties) | Value |
|---|---|
| Kinetic solubility@ | 361.0 μM (148.8 μg/ml) |
| Equilibrium solubility | 6.7 μM (2.76 μg/ml) |
| Log P | 3.18 |
| pKa | 11.33 |
| PAMPA | -4.49 |
| Plasma stability | >99.9 (Rat), 98.9 (Human) |
| Plasma protein binding | 90.2(Rat), 98.7 (Human) |

As is apparent from Table 3, the adenosine derivative of the inventive concept has absorption, distribution, metabolism and excretion (ADME) properties suitable for oral administration through an oral agent.

**<Experimental Example 3~7> Pharmacokinetic Test for Oral Administration of the Adenosine Derivative of the Inventive Concept**

In order to test the ADME properties of the adenosine derivative of the inventive concept after oral administration, pharmacokinetic properties of the compound of the formula A were measured in vivo.

As shown in Table 4, the compound of the formula A was administered to experimental animals using different administration methods. Intravenous administration was performed through a tube inserted into the femoral vein, and oral administration was performed using oral gavage.

[Table 4]

| Experimental Experimental | Example Animal | Administration Method |
|---|---|---|
| 3-1 | 8 week old SD male rat | Intravenous administration of male rat 5 mg/kg of the compound of the formula A |
| 3-2 | 8 week old SD male rat | Oral administration of 5 mg/kg of male rat the compound of the formula A |
| 4-1 | 8 week old SD male rat | Intravenous administration of male rat 2 mg/kg of the compound of the formula A |
| 4-2 | 8 week old SD male rat | Oral administration of 10 mg/kg of male rat the compound of the formula A |
| 5 | 8 week old ICR male mice | Oral administration of 10 mg/kg of male mice the compound of the formula A |
| 6-1 | Dog | Intravenous administration of 2 mg/kg of the compound of the formula A |
| 6-2 | Dog | Oral administration of 10 mg/kg of the compound of the formula A dissolved in a solvent |
| 6-3 | Dog | Oral administration of 10 mg/kg of the compound of the formula A contained in a powder state in a capsule |

(continued)

| Experimental Experimental | Example Animal | Administration Method |
|---|---|---|
| 7-1 | 8 week old SD male rat | Oral administration of 10 mg/kg of male rat the compound of the formula A dissolved in 2 mL/kg of 0.5% methyl cellulose |
| 7-2 | 8 week old SD male rat | Oral administration of 10 mg/kg of male rat the compound of the formula A dissolved in a solvent of a mixture of 5% DMSO, 40% PEG400 and 55% DW |

After the administration, the experimental animals' blood was taken at predetermined time intervals for 24 hours. Then, the blood was centrifuged to separate plasma. The plasma samples were pretreated with a suitable organic solvent, and then the concentration of the plasma samples was analyzed by LC-MS/MS. The blood concentration-time data of the compound of the formula A was analyzed using WinNonlin (Pharsight, USA), and graphs of the blood concentration-time data are shown in FIGS. 5 through 9. The results of noncompartmental pharmacokinetic parameters calculated from the blood concentration-time data are shown in Tables 5 through 9. In FIGS. 5 through 9, I.V. represents an intravenous administration group, P.O. represents an oral administration group, and the definition of each parameter in Tables 5 through 9 is shown in Table 10.

[Table 5]

| Parameters | I.V., 5 mg/kg | P.O., 5 mg/kg |
|---|---|---|
| $T_{max}$ (h) | NA | 1.33 ± 0.577 |
| $C_{max}$ (μg/mL) | NA | 1.45 ± 0.255 |
| $T_{1/2}$ (h) | 3.6 ± 0.589 | 3.26 ± 0.945 |
| $AUC_t$ (μg·h/mL) | 14.04 ± 2.55 | 6.98 ± 0.584 |
| $AUC_\infty$ (μg·h/mL) | 14.11 ± 2.59 | 7.04 ± 0.551 |
| CL (L/h/kg) | 0.363 ± 0.07 | NA |
| $V_{ss}$ (L/kg) | 0.881 ± 0.203 | NA |
| $F_t$ (%) | NA | 49.74 |

NA, not applicable; ND, not detected; NC, not calculated

[Table 6]

| Parameters | IV, 2 mg/kg | PO, 10 mg/kg |
|---|---|---|
| $T_{max}$ (hr) | - | 2.42 ± 3.13 |
| $C_{max}$ (μg/mL) | - | 2.71 ± 0.183 |
| $T_{1/2}$ (hr) | 6 ± 2.98 | 3.34 ± 0.075 |
| $AUC_t$ (μg·hr/mL) | 5.2 ± 0.548 | 26.5 ± 5.88 |
| $AUC_\infty$ (μg·hr/mL) | 5.49 ± 0.3 | 26.7 ± 0.0750 |
| CL (L/kg/hr) | 0.365 ± 0.019 | - |
| $V_{ss}$ (L/kg) | 2.27 ± 0.863 | - |
| $F_t$ (%) | - | >99.9 |

[Table 7]

| Parameters | P.O., 10 mg/kg |
|---|---|
| $T_{max}$ (h) | 6.13 ± 3.75 |
| $C_{max}$ (μg/mL) | 8.57 ± 1.52 |
| $T_{1/2}$ (h) | 3.61 ± 0.3 |
| $AUC_t$ (μg·h/mL) | 100 ± 13.2 |
| $AUC_\infty$ (μg·h/mL) | 102 ± 13.5 |
| CL (L/h/kg) | NA |
| $V_{ss}$ (L/kg) | NA |
| $F_t$ (%) | NA |

NA, not applicable; ND, not detected; NC, not calculated

[Table 8]

| Parameters | G1, IV, 2 mg/kg | G2, PO, 10 mg/kg | G3, PO, 10 mg/kg |
|---|---|---|---|
| $T_{max}$ (h) | NA | 1.67 ± 0.58 | 2 ± 0 |
| $C_{max}$ (μg/mL) | NA | 0.467 ± 0.073 | 1.14 ± 0.23 |
| $T_{1/2}$ (h) | 2.17 ± 0.867 | 4.21 ± 1.41 | 5.53 ± 3.06 |
| $AUC_t$ (μg·h/mL) | 0.948 ± 0.464 | 3.88 ± 1.03 | 5.64 ± 0.84 |
| $AUC_\infty$ (μg·h/mL) | 1.07 ± 0.62 | 3.99 ± 1.09 | 6.35 ± 0.83 |
| CL (L/h/kg) | 2.27 ± 1.04 | NA | NA |
| $V_{ss}$ (L/kg) | 6.02 ± 0.79 | NA | NA |
| $F_t$ (%) | NA | 82.0 | >99.9 |

NA, not applicable; ND, not detected; NC, not calculated

[Table 9]

| Parameters | 0.5%MC, 10 mg/kg | Conventional Vehicle, 10 mg/kg |
|---|---|---|
| $T_{max}$ (hr) | 1.33 ± 0.58 | 2.42 ± 3.13 |
| $C_{max}$ (μg/mL) | 5.72 ± 6.11 | 2.71 ± 0.183 |
| $T_{1/2}$ (hr) | 4.56 ± 2.8 | 3.34 ± 0.075 |
| $AUC_t$ (μg·hr/mL) | 40.1 ± 26.8 | 26.5 ± 5.88 |
| $AUC_\infty$ (μg·hr/mL) | 41.4 ± 26.03 | 26.7 ± 0.0750 |
| CL (L/kg/hr) | - | - |
| $V_{ss}$ (L/kg) | - | - |
| $F_t$ (%) | - | - |

[Table 10]

| Parameters | Description |
|---|---|
| $T_{max}$ (hr) | time for Cmax |
| $C_{max}$ (μg/mL) | maximum plasma concentration |
| T1/2 (hr) | terminal half-life |
| $AUC_t$ (μg·hr/mL) | areas under the plasma concentration-time curve |
| $AUC_\infty$ (μg·hr/mL) | areas under the plasma concentration-time curve from time |
| CL (L/kg/hr) | total clearance from plasma |
| $V_{ss}$ (L/kg) | steady-state volume of distribution |
| $F_t$ (%) | bioavailability ($AUC_{P.O}$/$AUC_{I.V.}$) X 100 |

FIG. 5 and Table 5 show a graph and parameter values obtained from the blood concentration-time data of Experimental Example 3 (3-1 and 3-2), and FIG. 6 and Table 6 show a graph and parameter values obtained from the blood concentration-time data of Experimental Example 4 (4-1 and 4-2). Referring to FIGS. 5 and 6 and Tables 5 and 6, the adenosine derivative of the inventive concept has a long half-life T1/2 of a maximum of 3.34 hours or more and a bioavailability Ft of a maximum of 99.9% or more in the case of oral administration. Thus, the adenosine derivative of the inventive concept is more suitable for oral administration than for intravenous administration.

FIG. 7 and Table 7 show a graph and parameter values obtained from the blood concentration-time data of Experimental Example 5. Referring to FIG. 7 and Table 7, the adenosine derivative of the inventive concept also has a long half-life T1/2 of about 3.61 hours in mice in the case of oral administration. Thus, the adenosine derivative is suitable for oral administration.

FIG. 8 and Table 8 show a graph and parameter values obtained from the blood concentration-time data of Experimental Example 6 (6-1, 6-2 and 6-3). In FIG. 8, G2 and G3 respectively indicate the oral administration of the adenosine derivative of the inventive concept dissolved in a solvent and the oral administration of the adenosine derivative of the inventive concept contained in a powder state in a capsule. Referring to FIG. 8 and Table 8, the adenosine derivative of the inventive concept has a longer half-life T1/2 of a maximum of 5.53 hours or more in dogs than in rats or mice. Thus, the adenosine derivative is suitable for oral administration. In particular, when the adenosine derivative of the inventive concept is administered in a powder state in a capsule, properties such as the half-life T1/2 and the bioavailability Ft are further improved.

FIG. 9 and Table 9 show a graph and parameter values obtained from the blood concentration-time data of Experimental Example 7 (7-1 and 7-2). Referring to FIG. 9 and Table 9, the adenosine derivative of the inventive concept exhibits better properties when orally administered together with methylcellulose (MC) than when orally administered together with conventional vehicles such as dimethyl sulfoxide (DMSO), polyethylene glycol (PEG) and distilled water (DW).

**Experimental Example 8: Toxicity Test of the Adenosine Derivative of the Present Invention**

The Adenosine Derivative of the present invention were assayed for cytotoxicity in animals. Three test groups of three 25±5 g ICR mice (Central Lab. Animal Inc., Korea) and three test groups of three 235±10 g specific pathogen-free (SPF) Sprague Dawley rats (Central Lab Animal Inc., Korea) were intraperitoneal injection with the compound of the formula A at doses of 20 mg/kg, 10 mg/kg, and 1 mg/kg, respectively, followed by observation for 24 hrs.

No death was observed in all three groups. No difference in weight gain or feed intake was detected between the control group and the test groups. Therefore, the derivative compounds of the present invention were proven as being safe.

In order to test the toxicity of the adenosine derivative of the present invention, the compound of the formula A was evaluated for cytotoxicity, hERG ligand binding assay, genotoxicity and single-dose toxicity.

First, a Cyto X™ cell viability assay kit was used to test the cytotoxicity of the compound of the formula A. According to the test results, the compound of the formula A had an IC50 of 10 μM or more in each cell line. Thus, the compound of the formula A was evaluated as safe in terms of general cytotoxicity.

In order to test the hERG ligand binding assay of the compound of the formula A, a non-electrophysiological method was used to evaluate heart stability by evaluating fluorescence polarization according to the degree of hERG channel protein binding of a red fluorescent hERG channel ligand tracer. According to the test results, an inhibition rate

for 10 μM of the compound of the formula A was 50% or less, i.e., a standard value. Thus, the compound of the formula A was evaluated as safe in terms of the hERG ligand binding assay.

In order to test the genotoxicity of the compound of the formula A, the gene mutagenicity of the compound of the formula A was evaluated in the presence and absence of metabolic activation by using histidine-requiring Salmonella (strains TA98, TA100, TA1535 and TA1537) and tryptophan-requiring Escherichia coli (strain WP2uvrA (pKM101)). According to the evaluation results, the number of back-mutant colonies of the compound of the formula A did not exceed twice the number of back-mutant colonies of the negative control group for all doses of each strain regardless of the metabolic activation, and no dose-dependent increase was observed in the compound of the formula A. In addition, for each strain, the number of back-mutant colonies in the positive control group certainly exceeded twice the number of back-mutant colonies in the negative control group. From the above results, the compound of the formula A was evaluated as safe in terms of genotoxicity.

In order to test the single-dose toxicity of the compound of the formula A, a single dose of 2,000 mg/kg of the compound of the formula A was administered to each of five male rats and five female rats. As a result of the test, no animals died. Thus, the compound of the formula A was evaluated as safe in terms of single-dose toxicity.

Table 11 summarizes the above toxicity test results of the adenosine derivative of the inventive concept. As is apparent from Table 11, the adenosine derivative of the inventive concept is safe in terms of cytotoxicity, hERG ligand binding assay, genotoxicity and single-dose toxicity.

[Table 11]

| **Test Type** | **Toxicity** |
|---|---|
| Cytotoxicity evaluation | Not detected |
| hERG ligand binding assay evaluation | Not detected |
| Genotoxicity evaluation | Not detected |
| Single-dose toxicity evaluation | Not detected |

## Experimental Example 9: Evaluation of Stability of an Oral Administration Agent Comprising Adenosine Derivatives of Present Invention

In order to evaluate the stability of the oral administration agent comprising the adenosine derivative of the present invention, the following experiment was performed.

0.5 wt % methylcellulose, which can be used as an excipient for oral administration, was added to the compound of the formula A, sonicated and homogenized, and then divided into a group to be stored at room temperature or at 4° C., and after 1, 3, 7, and 10 days, the stability was measured by comparing the concentration with 0.5% methyl cellulose as the control group using Waters UPLC and he results are shown in Table 12.

[Table 12]

| **Storage condition** | | **Stability (%)** | | |
|---|---|---|---|---|
| | | 200 μg/ml | 1000 μg/ml | 3000 μg/ml |
| **Control** | | 100±28.7 | 100±9.55 | 100±5.40 |
| Stored at RT | After 1 day | 84.3±4.10 | 82.7±16.3 | 90.5±6.44 |
| | After 3 days | 76.3±8.12 | 82.7±37.0 | 80.5±5.01 |
| | After 7 days | 80.9±14.6 | 75.4±6.66 | 101±13.2 |
| | After 10 days | 90.3±23.2 | 94.2±9.17 | 92.2±2.17 |
| Stored at 4°C | After 1 day | 118±21.4 | 76.5±11.7 | 84.8±17.9 |
| | After 3 days | 125±40.2 | 87.6±9.26 | 94.8±2.55 |
| | After 7 days | 88.3±17.3 | 80.1±27.7 | 89.7±8.30 |
| | After 10 days | 93.8±44.8 | 79.5±22.0 | 99.0±3.87 |

As shown in Table 12, when the adenosine derivative of the present invention was prepared as an oral administration agent, the stability of the adenosine derivative was not significantly different according to storage conditions

and storage time, and thus, it is confirmed that the adenosine derivative of the present invention was suitable for oral administration by an oral preparation.

**Experimental Example 10: Assay for Binding Affinity for Adenosine Receptors**

The adenosine derivatives of the present invention were assayed for binding affinity and selectivity for A1, A2A and A3 receptors among human adenosine receptor (hAR) as follows.

CHO cells (ATCC No. CCL-61), in which $A_1$ and $A_3$ adenosine receptors were expressed, were cultured in F-12 media (Gibco, U.S.A.) supplemented with 10% fetal bovine serum (FBS) and penicillin/streptomycin (100 units/ml and 100 g/ml), at 37° C. in a 5% $CO_2$ atmosphere. A predetermined amount of suitable hAR-expressed CHO cells was mixed with labeled ligands (1 nM $[^3H]$CCPA and 0.5 nM $[^{125}I]$AB-MECA) specifically binding to $A_1$ and $A_3$ adenosine receptors in a 50/10/1 buffer in test tubes. The derivatives of the present invention were dissolved at various concentrations in dimethyl sulfoxide (DMSO) and diluted in the buffer, taking care that the final concentration of DMSO did not exceed 1%. Incubation for 1 hr in a 37° C. incubator was followed by rapid filtration in a vacuum using a cell collector (TOMTEC, U.S.A.). Subsequently, the test tubes were washed three times with 3 ml of the buffer before radioactivity was measured using a $\gamma$-counter. In the same condition as that for total binding, the equilibrium constant $K_i$ for non-specific binding was determined in the presence of 10 $\mu$M of 5'-N-ethylcarboxamidoadenosine (NECA) as a non-labeled ligand. The equilibrium constant $K_i$ was calculated according to the Cheng-Prusoff equation on the assumption that $[^{125}I]$AB-MECA has a $K_d$ value of 1.48 nM. $K_i$ for binding affinity was determined by subtracting the non-specific binding value from the total binding value. On the basis of the specific binding values, the samples were analyzed for binding affinity to various adenosine receptors.

In addition, the binding of the labeled ligand $[^3H]$CGS-21680 (2-(((4-(2-carboxyethyl)phenyl)ethylamino)-5'-N-ethylcarbamoyl)adenosine) to the $A_{2A}$ adenosine receptor expressed on HEK 293 cell (human embryonic kidney cell grown in tissue culture) was assayed as follows. Adenosine deaminase was added alone or in combination with a radioactive ligand when cerebral meninges were incubated at 30° C. for 30 min. Each of the compounds synthesized in the examples was measured for $IC_{50}$ at least 6 different concentrations, and the measurements were analyzed using SigmaPlot software to determine $K_i$ values. Chemical Structures of the compounds synthesized in the examples, substituents, and $K_i$ values for binding affinity are summarized in Table 13, below.

[Table 13]

| **Ex. No.** | **Substituents** | | | **$K_i$(nM) or %** | | |
|---|---|---|---|---|---|---|
| | **A** | **R** | **Y** | **$hA_1$** | **$hA_{2A}$** | **$hA_3$** |
| 1 | S | 3- fluorobenzyl | Cl | 19.8% | 47.6% | 7.4 ± 1.3 |
| 2 | S | 3- chlorobenzyl | Cl | 37.9% | 17.7% | 1.66 ± 0.90 |
| 3 | S | 3- bromobenzyl | Cl | 34.2% | 18.4% | 8.99 ± 5.17 |
| 4 | S | 3- iodobenzyl | Cl | 2490 ± 940 | 341 ± 75 | 4.16 ± 0.50 |
| 5 | S | 2- chlorobenzyl | Cl | 12.8% | 1600 ± 135 | 25.8 ± 6.3 |
| 6 | S | 5-chloro-2-methoxybenzyl | Cl | 23.8% | 4020 ± 1750 | 12.7 ± 3.7 |
| 7 | S | 2- methoxybenzyl | Cl | 9.4% | 17.5% | 19.9 ± 7.1 |
| 8 | S | 1- naphthylmethyl | Cl | 22.0% | -8.3% | 24.8 ± 8.1 |
| 9 | S | 3- toluic acid | Cl | 13.1% | -0.18% | 41.5% |
| 10 | S | methyl | Cl | 55.4 ± 1.8% | 45.0 ± 1.4% | 3.69 ± 0.25 |
| 11 | S | 3- fluorobenzyl | H | 1430 ± 420 | 1260 ± 330 | 7.3 ± 0.6 |
| 12 | S | 3- chlorobenzyl | H | 860 ± 210 | 440 ± 110 | 1.5 ± 0.4 |
| 13 | S | 3- bromobenzyl | H | 790 ± 190 | 420 ± 32 | 6.8 ± 3.4 |
| 14 | S | 3- iodobenzyl | H | 530 ± 97 | 230 ± 65 | 2.5 ± 1.0 |
| 15 | O | 3- bromobenzyl | Cl | 39.8% | 22.8% | 13.0 ± 6.9 |
| 16 | O | 3- iodobenzyl | Cl | 37.7% | 28.6% | 42.9 ± 8.9 |

(continued)

| Ex. No. | Substituents | | | $K_i$(nM) or % | | |
|---|---|---|---|---|---|---|
| | A | R | Y | $hA_1$ | $hA_{2A}$ | $hA_3$ |
| Unit nM ± SEM<br>"%" represents percentage inhibition of specific binding of 10 μM labeled ligand in the presence of 10 μM of the unlabeled ligand NECA. | | | | | | |

<Chemical Formula 1>

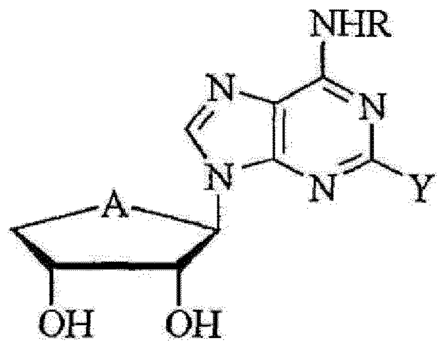

As can be understood from the data of Table 1, the compounds synthesized in the examples of the present invention were found to have high binding affinity for human A3 adenosine receptors (hA3AR), but low affinity for A1 and A2A adenosine receptors, thereby showing high selectivity.

## Experimental Examples 11 to 14: Anti-Inflammatory Activity of Adenosine Derivatives

The adenosine derivatives of the present invention were examined for anti-inflammatory activity in the following animal test. Seven-week-old male ICR mice were treated with TPA (12-O-tetradecanoylphorbol-13-acetate, 20 μl) in the right ear. Within 15 minutes, the compounds of Examples 1 to 16 were diluted at a concentration of 0.5% in acetone (20 μl), distilled water, or mixtures of DMSO and acetone (compositions shown in Tables 14 to 17) before being administered to the mice. Hydrocortisone was used at the same concentration as a control.

6 hrs after treatment with TPA, the mice were secondarily treated with the adenosine derivatives of the present invention. 24 hrs after TPA treatment, test animals were euthanized using a cervical dislocation method. Samples were obtained from the right ear using a 6 mm diameter punch. The activity was observed by measuring the ear sample using a microbalance. Percentages of inhibition were calculated using the following Equation 1. The compositions and amounts used in these experiments are summarized in Tables 14 to 17 and the anti-inflammatory activities thereof are shown in FIGS. 10 to 13.

$$\%\ \text{Inhibition} = \frac{1 - Rt.\text{Ear(Test-Non treated)}}{Rt.\text{Ear}(TPA\ \text{only-Non treated})} \qquad \text{[Equation 1]}$$

[Table 14]

| Exp. Ex. 11 | Compositions | Amounts |
|---|---|---|
| 11-1 | Non-treated | - |
| 11-2 | TPA alone | 20 μl |
| 11-3 | TPA + acetone | 20 μl + 20 μl |
| 11-4 | TPA + acetone + Cpd. Of Ex. 2 | 20 μl + 0.5% / 20 μl |
| 11-5 | TPA + acetone + Cpd. Of Ex. 3 | 20 μl + 0.5% / 20 μl |

(continued)

| Exp. Ex. 11 | Compositions | Amounts |
|---|---|---|
| 11-6 | TPA + acetone + Cpd. Of Ex. 4 | 20 μl + 0.5% / 20 μl |
| 11-7 | TPA + acetone + hydrocortisone | 20 μl + 0.5% / 20 μl |

[Table 15]

| Exp. Ex. 12 | Compositions | Amounts |
|---|---|---|
| 12-1 | Non-treated | - |
| 12-2 | TPA alone | 20 μl |
| 12-3 | TPA + acetone | 20 μl + 20 μl |
| 12-4 | TPA + acetone + Cpd. Of Ex. 1 | 20 μl + 0.5% / 20 μl |
| 12-5 | TPA + acetone + Cpd. Of Ex. 6 | 20 μl + 0.5% / 20 μl |
| 12-6 | TPA + acetone + hydrocortisone | 20 μl + 0.5% / 20 μl |

[Table 16]

| Exp. Ex. 13 | Compositions | Amounts |
|---|---|---|
| 13-1 | Non-treated | - |
| 13-2 | TPA alone | 20 μl |
| 13-3 | TPA + solvent mix (water: acetone 1:4) | 20 μl + 20 μl |
| 13-4 | TPA + solvent mix + Cpd. Of Ex. 5 | 20 μl + 0.5% / 20 μl |
| 13-5 | TPA + solvent mix + Cpd. Of Ex. 7 | 20 μl + 0.5% / 20 μl |
| 13-6 | TPA + solvent mix + Cpd. Of Ex. 8 | 20 μl + 0.5% / 20 μl |
| 13-7 | TPA + solvent mix + hydrocortisone | 20 μl + 0.5% / 20 μl |

[Table 17]

| Exp. Ex. 14 | Compositions | Amounts |
|---|---|---|
| 14-1 | Non-treated | - |
| 14-2 | TPA alone | 20 μl |
| 14-3 | TPA + solvent mix (DMSO:acetone 1:9) | 20 μl + 20 μl |
| 14-4 | TPA + solvent mix + Cpd. Of Ex. 15 | 20 μl + 0.5% / 20 μl |
| 14-5 | TPA + solvent mix + Cpd. Of Ex. 16 | 20 μl + 0.5% / 20 μl |
| 14-6 | TPA + solvent mix + hydrocortisone | 20 μl + 0.5% / 20 μl |

When applied to the mice, as seen in FIG. 10, dilutions of the compounds of Examples 2 to 4 were found to inhibit the TPA-induced inflammation of the mouse ear to some degree.

The anti-inflammatory activity of the compounds of Examples 1 and 6, as shown in FIG. 11, was measured to be increased.

As seen in FIG. 12, the compounds of Examples 5 to 7, diluted at a concentration of 0.5% in a mixture of distilled water and acetone (1:4), were measured to have percentages of inflammation inhibition of 17%, 34% and 53%, respectively.

As shown in FIG. 13, the compounds of Examples 15 and 16, diluted at a concentration of 0.5% in a mixture

of DMSO and acetone (1:9), were measured to have percentages of inflammation inhibition of 59% and 79%, respectively. Based on the observations in this test, the adenosine derivatives of the present invention were proven to have anti-inflammatory activity.

As described above, although the present invention is mainly described with reference to the embodiments of the present invention, this is merely an example and does not limit the present invention, and it will be appreciated that a person with ordinary skill in the art to which the present invention pertains can make various modifications and applications which are not exemplified above within a range not departing from the essential characteristics of the embodiments of the present invention. For example, each constituent element specifically shown in the embodiments of the present invention can be modified and implemented. And differences related to these modifications and applications should be construed as being included in the scope of the present invention defined in the appended claims.

## Claims

1. A pharmaceutical composition for use in preventing and/or treating cholangitis or a liver disease caused by cholangitis, comprising a compound represented by the following Chemical Formula 1 or a pharmaceutically acceptable salt thereof; and

   a pharmaceutically acceptable carrier.

<Chemical Formula 1>

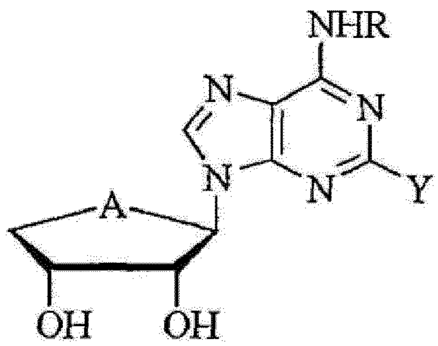

(wherein,
A is O or S,
R is an unsubstituted $C_1$ to $C_5$ alkyl; a $C_1$ to $C_5$ alkyl substituted with at least one $C_6$ to $C_{10}$ aryl; unsubstituted benzyl; benzyl substituted with one or more selected from the group consisting of a halogen and a $C_1$ to $C_4$ alkoxy; or benzyl substituted with hydroxycarbonyl), and
Y is H or a halogen element.)

2. The pharmaceutical composition of claim 1, wherein in Chemical Formula 1,

   A is O or S,
   R is methyl; ethyl; propyl; napthylmethyl; benzyl; benzyl substituted with one or more selected from the group consisting of a halogen and a $C_1$ to $C_3$ alkoxy; or toluic acid, and
   Y is H or Cl.

3. The pharmaceutical composition of claim 2, wherein in Chemical Formula 1, Ais S,

R is methyl, ethyl, 1-naphthylmethyl, benzyl, 2-chlorobenzyl, 3-fluorobenzyl, 3-chlorobenzyl, 3-bromobenzyl, 3-iodobenzyl, 2-methoxy-5-chlorobenzyl, 2-methoxybenzyl or 3-toluic acid, and
Y is Cl.

4. The pharmaceutical composition of claim 3, wherein Chemical Formula 1 is the following formula A.

<formula A>

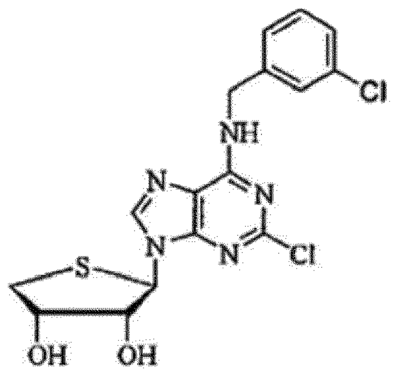

5. The pharmaceutical composition of claim 1, wherein the cholangitis is a cholestatic disease (cholestasis).

6. The pharmaceutical composition of claim 5, wherein the cholestatic disease comprises one or more selected from the group consisting of primary biliary cholangitis (PBC), primary sclerosing cholangitis (PSC), cholecystitis, drug induced cholestasis, post-liver transplantation cholestasis, progressive familial intrahepatic cholestasis (PFIC), Alagille Syndrome (ALGS), biliary atresia, intrahepatic cholestasis of pregnancy (ICP), cholelithiasis, infectious cholangitis, cholangitis associated with Langerhans cell histiocytosis, non-syndromic ductal paucity and total parenteral nutrition-associated cholestasis.

7. The pharmaceutical composition of claim 6, wherein the cholestatic disease further comprises a complication caused by the cholestatic disease, and the complication comprises one or more selected from the group consisting of pruritus, hypercholesterolemia, Sicca syndrome, Raynaud syndrome, osteoporosis, ulcerative colitis and colorectal cancer.

8. The pharmaceutical composition of claim 1, wherein the disease caused by cholangitis comprises one or more of liver fibrosis, liver cirrhosis and hepatitis.

9. The pharmaceutical composition of claim 1, wherein it is possible to lower the values of one or more of AST, ALT, AAR, hyaluronic acid and hydroxyproline in a liver tissue or blood of an individual to which the composition is administered.

10. The pharmaceutical composition of claim 1, wherein the pharmaceutical composition is formulated as an oral administration agent.

11. The pharmaceutical composition of claim 10, wherein the oral administration agent further comprises methyl cellulose (MC).

12. The pharmaceutical composition of claim 10, wherein the oral administration agent is an oral administration agent in which the compound represented by Chemical Formula 1 or a pharmaceutically acceptable salt thereof is filled in a powder stat in a capsule.

[Fig. 1]

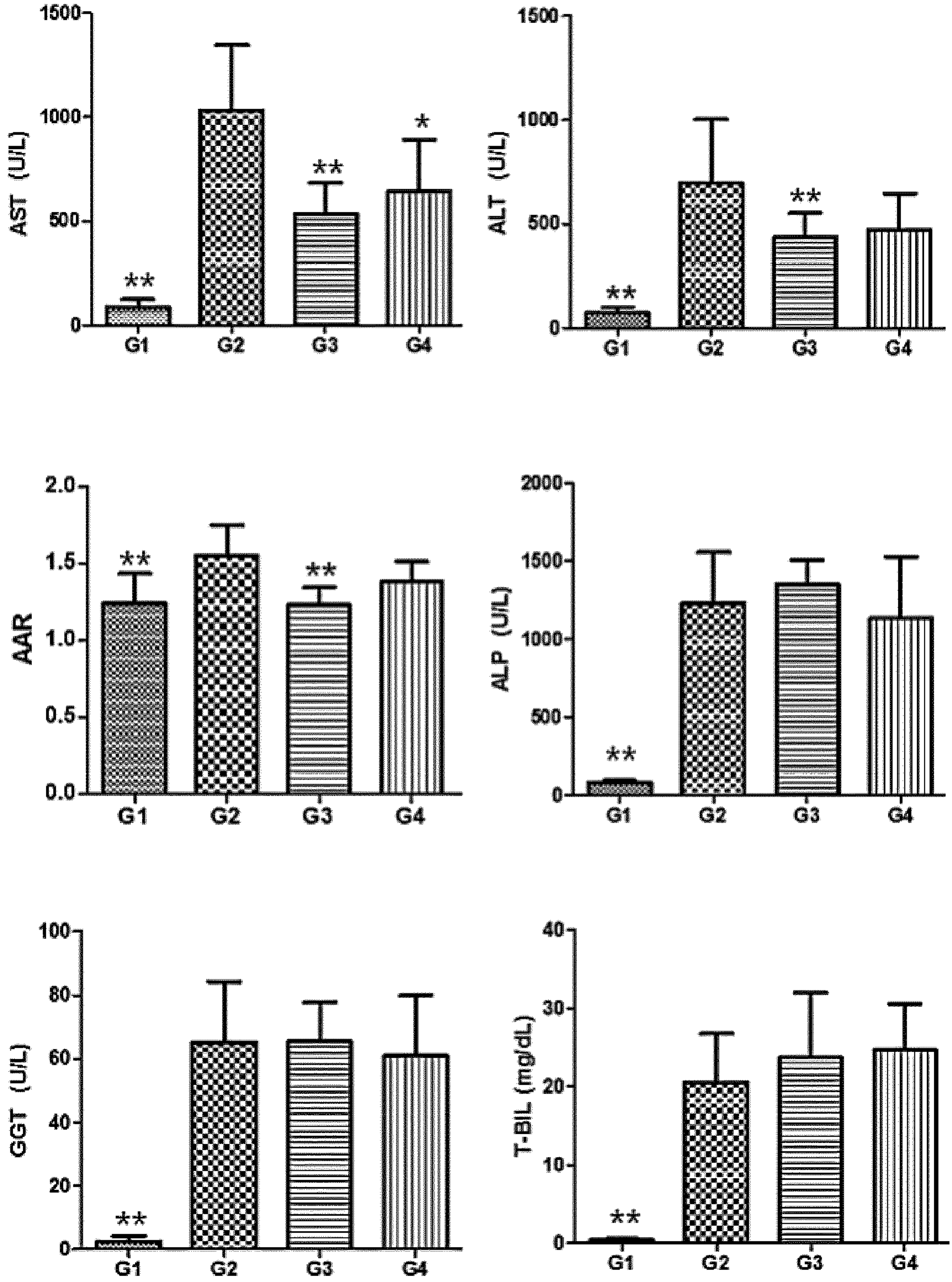
AST (U/L)
1500
1000
500
0
G1
G2
G3
G4
**
**
*
ALT (U/L)
1500
1000
500
0
G1
G2
G3
G4
**
**
AAR
2.0
1.5
1.0
0.5
0.0
G1
G2
G3
G4
**
**
ALP (U/L)
2000
1500
1000
500
0
G1
G2
G3
G4
**
GGT (U/L)
100
80
60
40
20
0
G1
G2
G3
G4
**
T-BIL (mg/dL)
40
30
20
10
0
G1
G2
G3
G4
**

[Fig. 2]

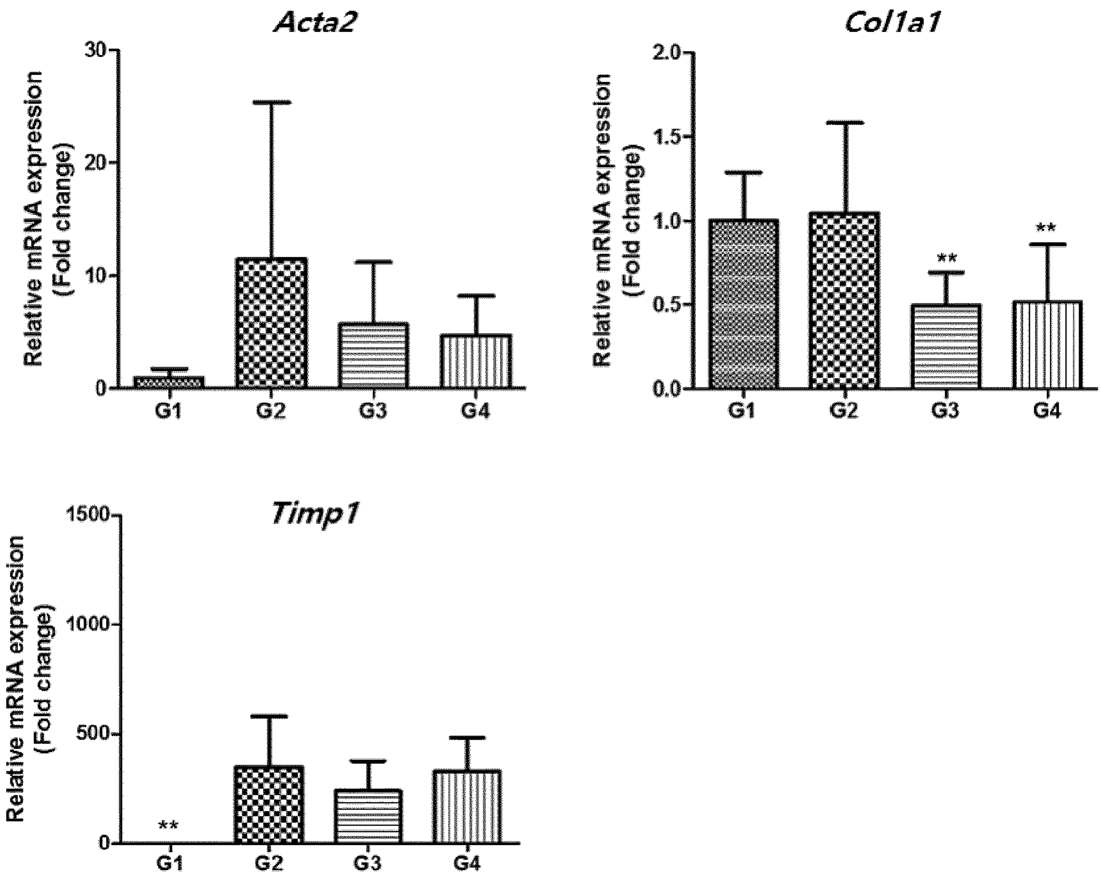
Acta2
Relative mRNA expression (Fold change)
30
20
10
0
G1
G2
G3
G4
Col1a1
Relative mRNA expression (Fold change)
2.0
1.5
1.0
0.5
0.0
**
**
G1
G2
G3
G4
Timp1
Relative mRNA expression (Fold change)
1500
1000
500
0
**
G1
G2
G3
G4

[Fig. 3]

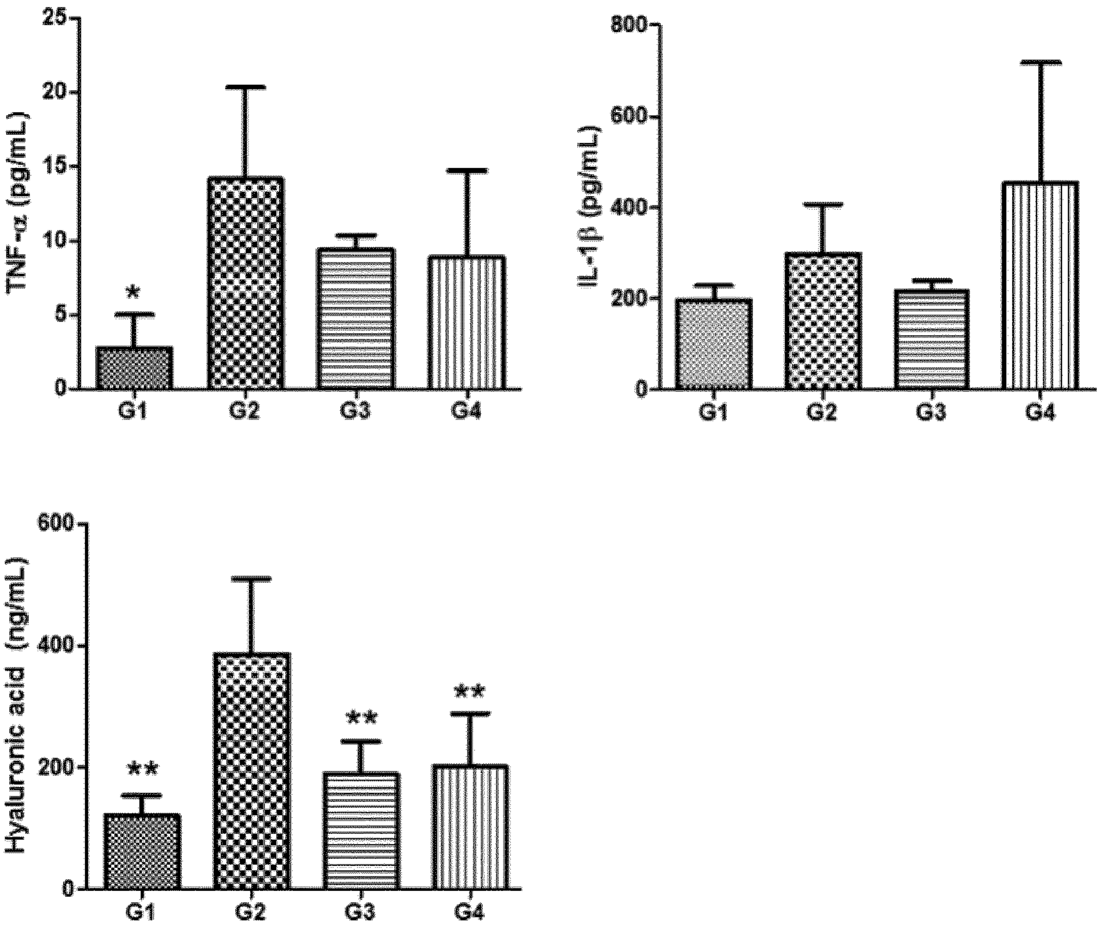
25
20
15
10
5
0
TNF-α (pg/mL)
*
G1
G2
G3
G4
800
600
400
200
0
IL-1β (pg/mL)
G1
G2
G3
G4
600
400
200
0
Hyaluronic acid (ng/mL)
**
**
**
G1
G2
G3
G4

[Fig. 4]

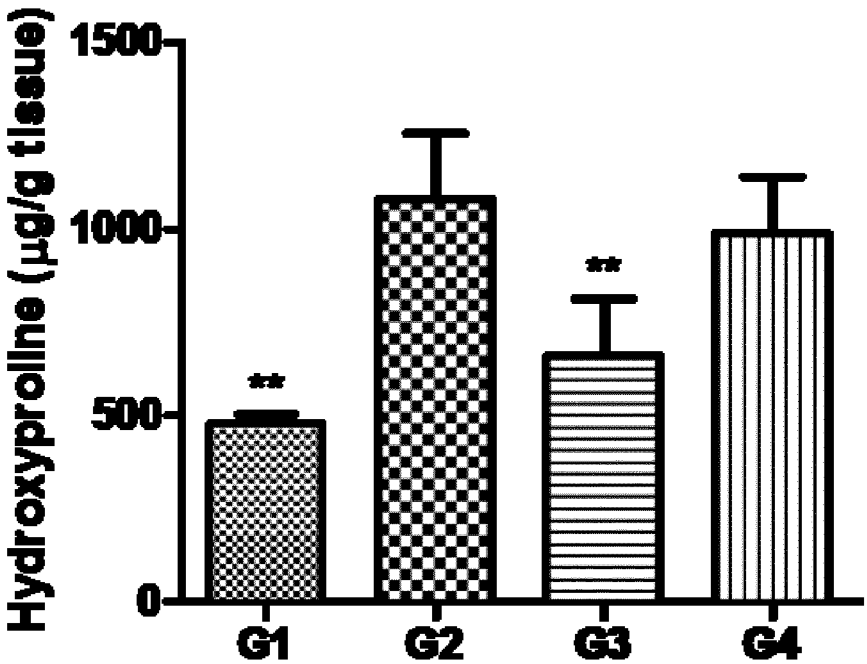

[Fig. 5]

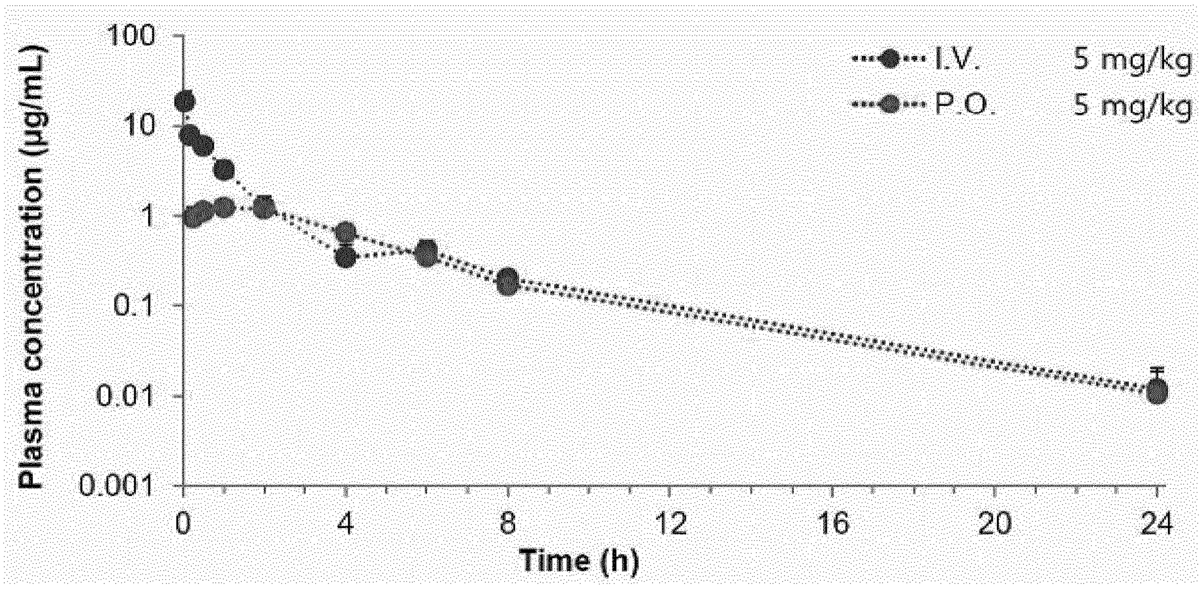
I.V. 5 mg/kg
P.O. 5 mg/kg
Plasma concentration (μg/mL)
100
10
1
0.1
0.01
0.001
0
4
8
12
16
20
24
Time (h)

[Fig. 6]

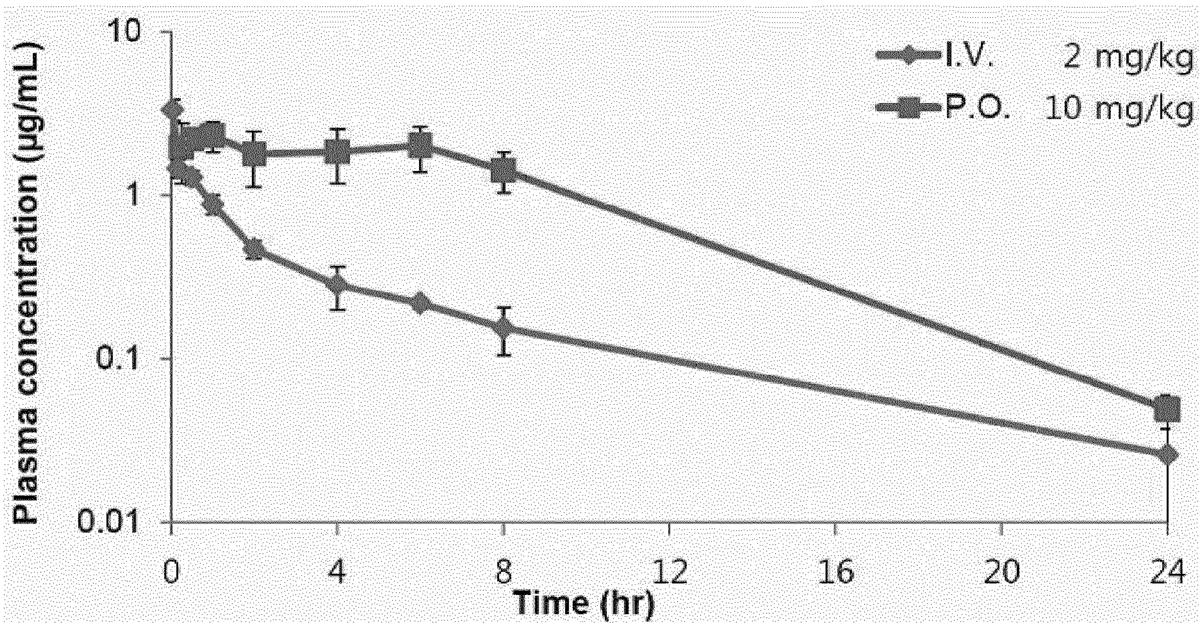
I.V. 2 mg/kg
P.O. 10 mg/kg
Plasma concentration (μg/mL)
10
1
0.1
0.01
0
4
8
12
16
20
24
Time (hr)

[Fig. 7]

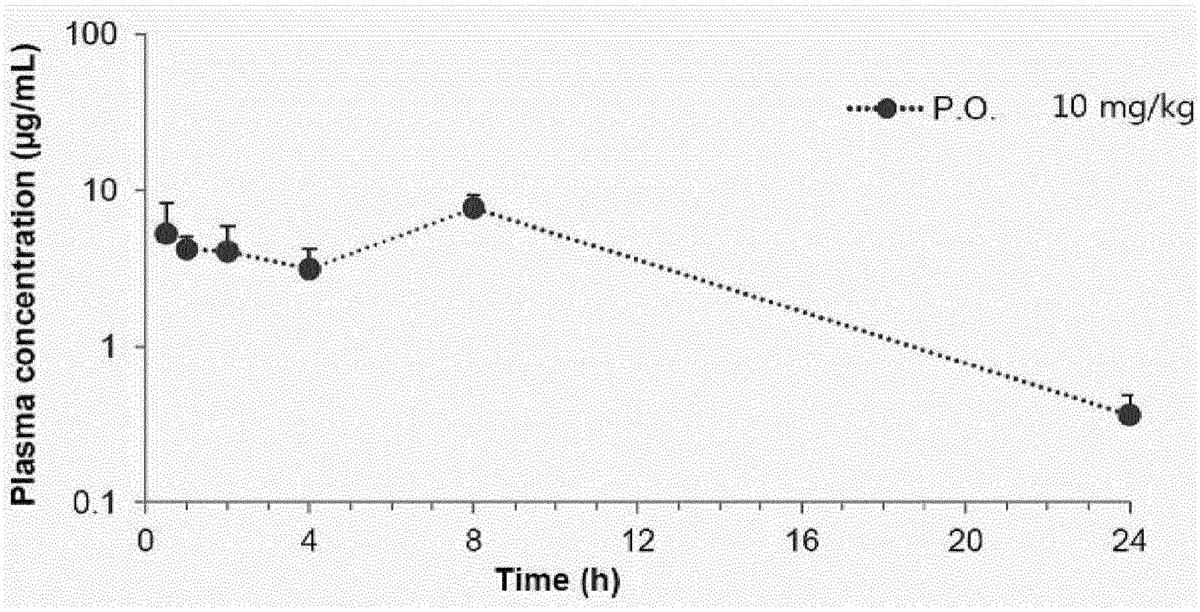
Plasma concentration (µg/mL)
100
10
1
0.1
P.O. 10 mg/kg
0
4
8
12
16
20
24
Time (h)

[Fig. 8]

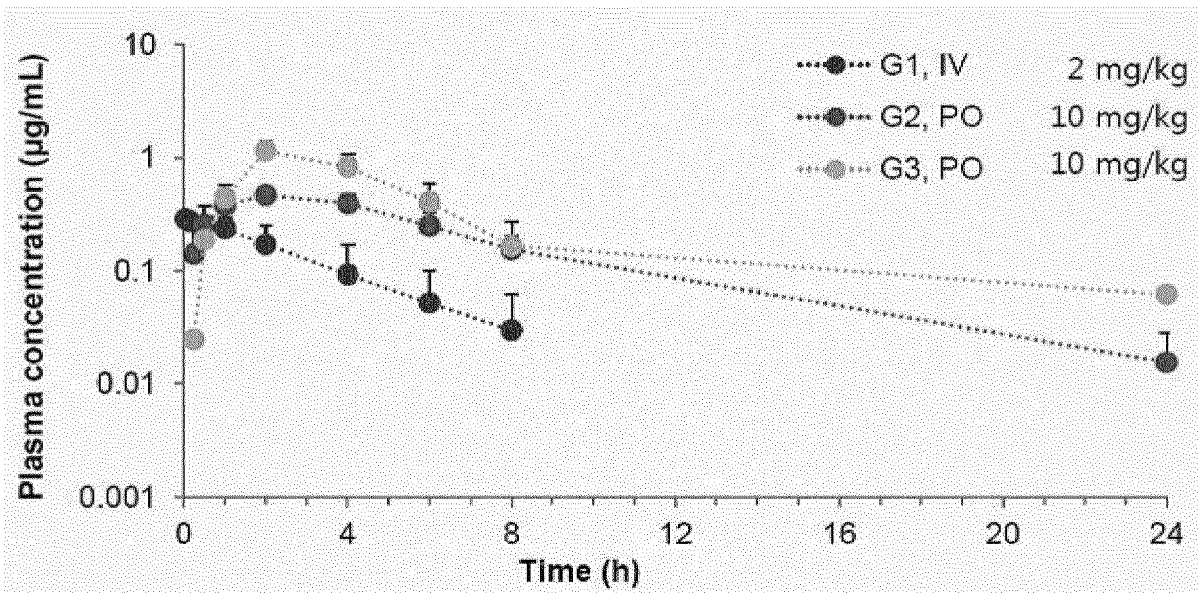
Plasma concentration (µg/mL)
10
1
0.1
0.01
0.001
G1, IV 2 mg/kg
G2, PO 10 mg/kg
G3, PO 10 mg/kg
0
4
8
12
16
20
24
Time (h)

[Fig. 9]

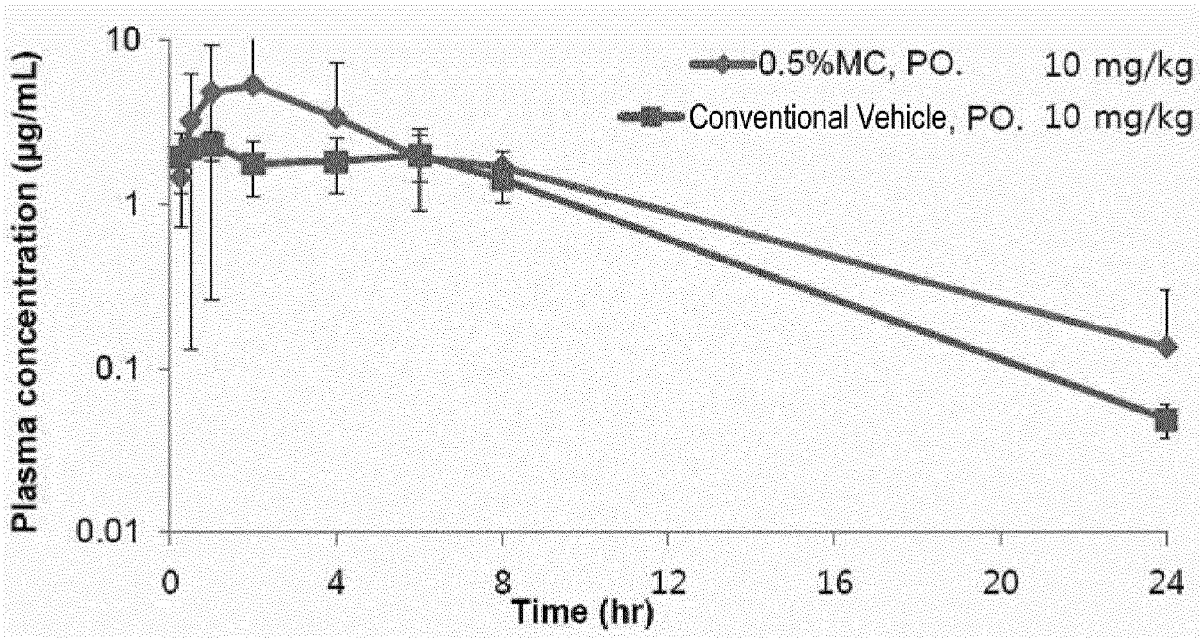
Plasma concentration (µg/mL)
10
1
0.1
0.01
0
4
8
12
16
20
24
Time (hr)
0.5%MC, PO. 10 mg/kg
Conventional Vehicle, PO. 10 mg/kg

[Fig. 10]

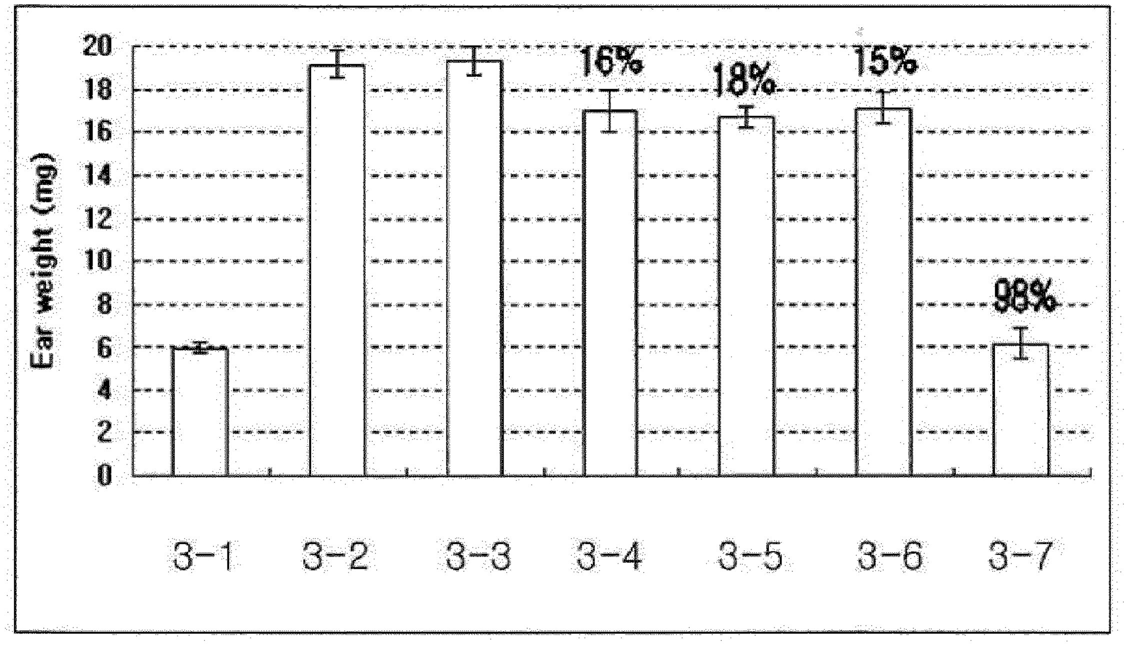
Ear weight (mg)
20
18
16
14
12
10
8
6
4
2
0
16%
18%
15%
98%
3-1
3-2
3-3
3-4
3-5
3-6
3-7

[Fig. 11]

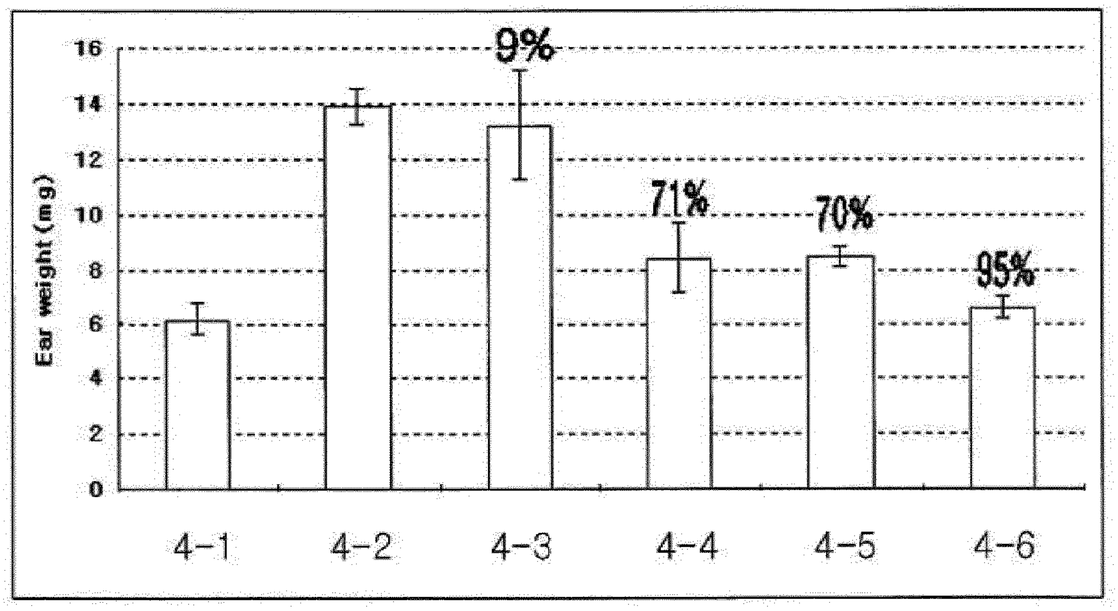
Ear weight (mg)
16
14
12
10
8
6
4
2
0
9%
71%
70%
95%
4-1
4-2
4-3
4-4
4-5
4-6

[Fig. 12]

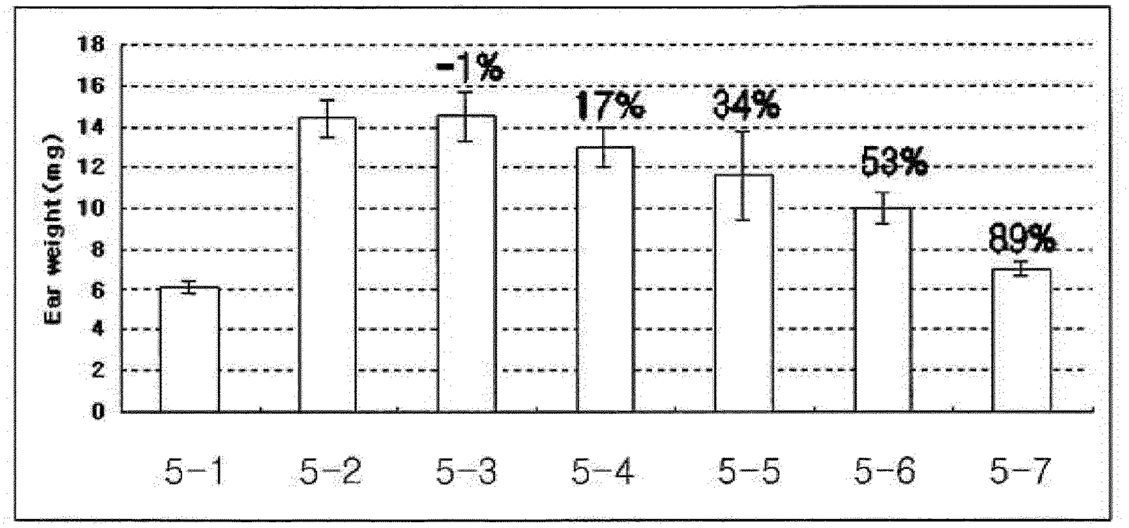
Ear weight (mg)
18
16
14
12
10
8
6
4
2
0
-1%
17%
34%
53%
89%
5-1
5-2
5-3
5-4
5-5
5-6
5-7

[Fig. 13]

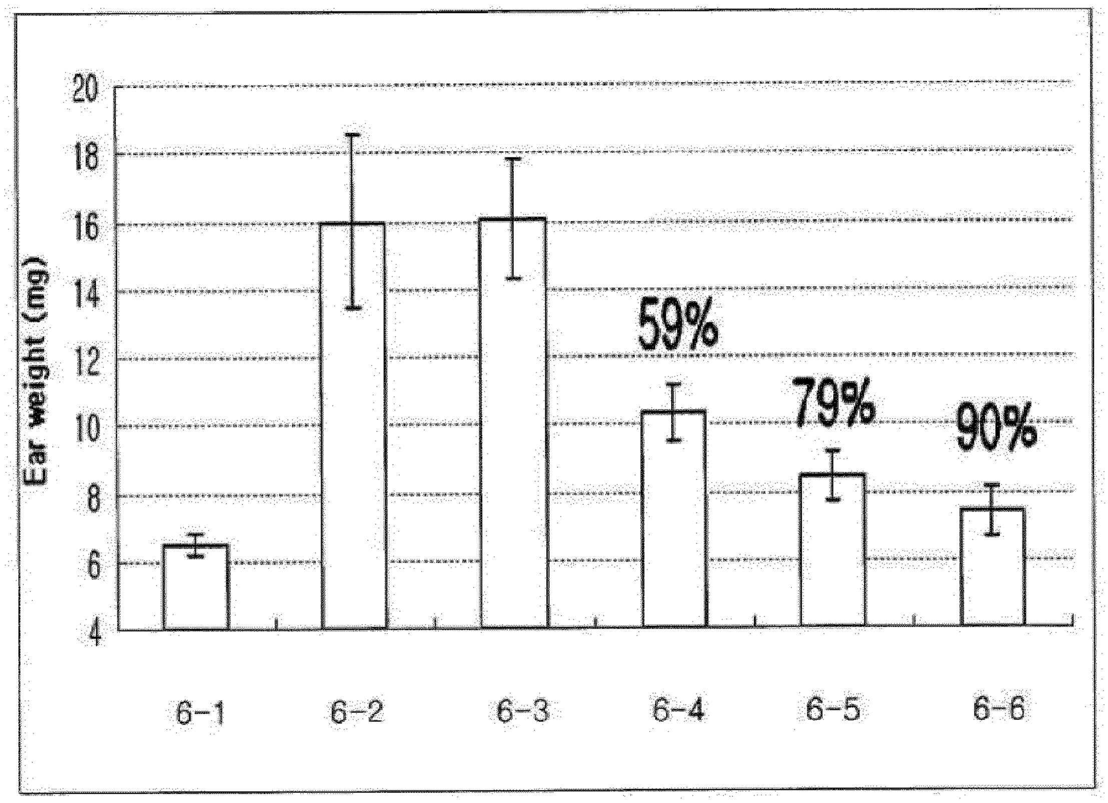
Ear weight (mg)
20
18
16
14
12
10
8
6
4
59%
79%
90%
6-1
6-2
6-3
6-4
6-5
6-6

Europäisches Patentamt
European Patent Office
Office européen des brevets

# EUROPEAN SEARCH REPORT

Application Number
EP 21 21 0303

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 533 452 A1 (FUTURE MEDICINE CO LTD [KR]) 4 September 2019 (2019-09-04)<br>* claims 1, 3, 4-6 *<br>* page 32, paragraph 144 - page 34, paragraph 149; examples 3-6 *<br>----- | 1-12 | INV.<br>A61K31/7076<br>A61K9/00<br>A61P1/16 |
| A | Lee Jae-Young ET AL: "Determination and validation of LJ-2698, a potent human A 3 adenosine receptor antagonist, in rat plasma by liquid chromatography-tandem mass spectrometry and its application in pharmacokinetic study In-Soo Yoon 4 @BULLET Lak Shin Jeong 1 @BULLET Dae-Duk Kim",<br>,<br>29 July 2017 (2017-07-29), pages 952-961, XP055916934,<br>Retrieved from the Internet:<br>URL:https://link.springer.com/content/pdf/10.1007/s12272-017-0935-9.pdf<br>[retrieved on 2022-05-02]<br>* abstract *<br>----- | 1-12 | |
| | | | TECHNICAL FIELDS SEARCHED (IPC)<br>A61K<br>A61P |
| A | EP 3 725 317 A1 (FUTURE MEDICINE CO LTD [KR]) 21 October 2020 (2020-10-21)<br>* claim 1 *<br>----- | 1-12 | |
| A | KR 101 881 441 B1 (SEOUL NAT UNIV R&DB FOUNDATION [KR]) 24 July 2018 (2018-07-24)<br>* abstract *<br>* claim 1 *<br>----- | 1-12 | |
| A | WO 2008/108508 A1 (FM THERAPEUTICS CO LTD [KR]; JEONG LAK SHIN [KR] ET AL.) 12 September 2008 (2008-09-12)<br>* page 1, line 7 - line 15 *<br>* claim 1 *<br>----- | 1-12 | |

The present search report has been drawn up for all claims

1

| Place of search | Date of completion of the search | Examiner |
|---|---|---|
| Munich | 5 May 2022 | Werner, Doris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 21 21 0303

This annex lists the patent family members relating to the patent documents cited in the above-mentioned European search report. The members are as contained in the European Patent Office EDP file on
The European Patent Office is in no way liable for these particulars which are merely given for the purpose of information.

05-05-2022

| Patent document cited in search report | Publication date | Patent family member(s) | Publication date |
|---|---|---|---|
| EP 3533452 A1 | 04-09-2019 | AU 2017349214 A1 | 20-06-2019 |
| | | CA 3042181 A1 | 03-05-2018 |
| | | CN 109906083 A | 18-06-2019 |
| | | CN 113616659 A | 09-11-2021 |
| | | EP 3533452 A1 | 04-09-2019 |
| | | JP 6912562 B2 | 04-08-2021 |
| | | JP 2019535659 A | 12-12-2019 |
| | | JP 2021073225 A | 13-05-2021 |
| | | KR 101709307 B1 | 23-02-2017 |
| | | TW 201828949 A | 16-08-2018 |
| | | US 2020046711 A1 | 13-02-2020 |
| | | WO 2018080127 A1 | 03-05-2018 |
| EP 3725317 A1 | 21-10-2020 | AU 2018374675 A1 | 18-06-2020 |
| | | CA 3083398 A1 | 06-06-2019 |
| | | CN 111432822 A | 17-07-2020 |
| | | EP 3725317 A1 | 21-10-2020 |
| | | JP 2021504494 A | 15-02-2021 |
| | | KR 20190062894 A | 07-06-2019 |
| | | US 2021361690 A1 | 25-11-2021 |
| | | WO 2019107964 A1 | 06-06-2019 |
| KR 101881441 B1 | 24-07-2018 | NONE | |
| WO 2008108508 A1 | 12-09-2008 | AU 2007348394 A1 | 12-09-2008 |
| | | CA 2680179 A1 | 12-09-2008 |
| | | CN 101801970 A | 11-08-2010 |
| | | EP 2142549 A1 | 13-01-2010 |
| | | JP 5306238 B2 | 02-10-2013 |
| | | JP 2010520271 A | 10-06-2010 |
| | | US 2010137577 A1 | 03-06-2010 |
| | | WO 2008108508 A1 | 12-09-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 101396092

### Non-patent literature cited in the description


- **LIEDTKE, C. et al.** Experimental liver fibrosis research: update on animal models, legal issues and translational aspects. *Fibrogenesis Tissue Repair,* 2013, vol. 6 (1), 19
- **LI, X. ; LIU, R. ; HUANG, Z. ; GURLEY, E.C. ; WANG, X. ; WANG, J. ; HE, H. ; YANG, H. ; LAI, G ; ZHANG, L.** Cholangiocyte-derived exosomal long noncoding RNA H19 promotes cholestatic liver injury in mouse and humans. *Hepatology,* 2018, vol. 68, 599-615
- **YOU LI ; RUQI TANG ; PATRICK S.C ; LEUNG, M. ; ERIC GERSHWIN ; XIONG MA.** Bile acids and intestinal microbiota in autoimmune cholestatic liver diseases. *Autoimmunity Reviews,* 2017, vol. 16 (9), ISSN 1568-9972, 885-896, https://doi.org/10.1016/j.autrev.2017.07.002
- **LLEO, A. ; MARONI, L. ; GLASER, S. ; ALPINI, G ; MARZIONI, M.** Role of cholangiocytes in primary biliary cirrhosis. *Semin Liver Dis,* 2014, vol. 34 (3), 273-284
- Primary Biliary Cholangitis. **CIVAN, J.** Merck Manual Professional Version. 2019
- **AGUILAR, M. T. ; CHASCSA, D. M.** Update on Emerging Treatment Options for Primary Biliary Cholangitis. *Hepat Med,* 2020, vol. 12, 69-77
- **TAG, C. G et al.** Bile Duct Ligation in Mice: Induction of Inflammatory Liver Injury and Fibrosis by Obstructive Cholestasis. *J. Vis. Exp.,* 2015, vol. 96, e52438
- *Am J Gastroenterol,* 2004, vol. 99, 1160-1174
- **ARJMAND, A. et al.** Quantification of Liver Fibrosis-A Comparative Study. *Appl. Sci.,* 2020, vol. 10, 447